(19) Europäisches Patentamt
European Patent Office
Office européen des brevets

(11) Veröffentlichungsnummer: **0 308 364 B1**

(12) **EUROPÄISCHE PATENTSCHRIFT**

(45) Veröffentlichungstag der Patentschrift: **10.11.93**

(51) Int. Cl.$^5$: **C07C 233/64**, A61K 49/04, C07C 231/00

(21) Anmeldenummer: **88730215.6**

(22) Anmeldetag: **15.09.88**

# (54) Neue Dicarbonsäure-bis(3,5-dicarbamoyl-2,4,6-Triiodanilide), Verfahren zu deren Herstellung sowie diese enthaltende Röntgenkontrastmittel.

(30) Priorität: **17.09.87 DE 3731542**

(43) Veröffentlichungstag der Anmeldung:
**22.03.89 Patentblatt 89/12**

(45) Bekanntmachung des Hinweises auf die Patenterteilung:
**10.11.93 Patentblatt 93/45**

(84) Benannte Vertragsstaaten:
**AT BE CH DE ES FR GB GR IT LI LU NL SE**

(56) Entgegenhaltungen:
**EP-A- 0 022 056**
**EP-A- 0 049 745**
**DE-A- 2 628 517**

(73) Patentinhaber: **SCHERING AKTIENGESELLSCHAFT Berlin und Bergkamen**
**Müllerstrasse 170/178**
**Postfach 65 03 11**
**D-13303 Berlin(DE)**

(72) Erfinder: **Speck, Ulrich, Prof. Dr.**
**Benediktiner Strasse 50**
**D-1000 Berlin 28(DE)**
Erfinder: **Blaszkiewicz, Peter, Dr.**
**Rothenburgstrasse 25**
**D-1000 Berlin 41(DE)**

**EP 0 308 364 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Dicarbonsäure-bis(3,5-dicarbamoyl-2,4,6-triiodanilide) der allgemeinen Formel I

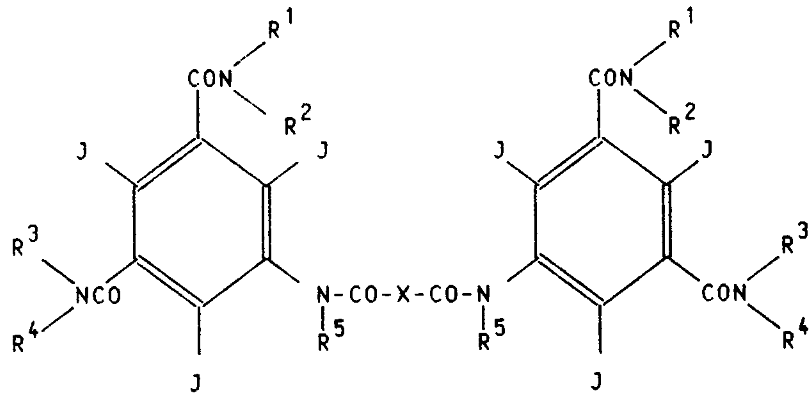

(I)

worin die Amidreste $-CONR^1R^2$ und $-CONR^3R^4$ voneinander verschieden sind und

- $R^1$ ein Wasserstoffatom, einen niederen Alkylrest oder $R^2$,
- $R^2$ einen gerad- oder verzweigtkettigen Mono- oder Polyhydroxyalkylrest,
- $R^3$ ein Wasserstoffatom, einen niederen Alkylrest oder $R^4$,
- $R^4$ einen gerad- oder verzweigtkettigen Mono- oder Polyhydroxyalkylrest,
- $R^5$ ein Wasserstoffatom, einen niederen Alkylrest oder einen Mono- oder Polyhydroxyalkylrest und
- X ein gerad- oder verzweigtkettiges Alkylen mit 1 bis 6 Kohlenstoffatomen, das gewünschtenfalls durch 1 bis 6 Hydroxy- oder Alkoxygruppen substituiert oder durch ein oder mehrere Sauerstoffatome unterbrochen sein kann,

bedeutet,
sowie ein Verfahren zur Herstellung von Röntgenkontrastmittel, die Verbindungen der Formel I als schattengebende Substanz enthalten.

Die Reste $R^1$ und $R^3$ enthalten als niedere Alkylreste vorzugsweise geradkettige Reste mit 1 bis 4 Kohlenstoffatomen, vorzugsweise mit 1 bis 2 Kohlenstoffatomen wie beispielsweise Ethyl, Propyl, Butyl, insbesondere Methyl.

Die Reste $R^2$ und $R^4$ enthalten als gerad- oder verzweigtkettige Mono- oder Polyhydroxyalkylreste 2 bis 8 Kohlenstoffatome, vorzugsweise 2 bis 5 Kohlenstoffatome. Geradkettige Reste von $R^2$ und $R^4$ bestehen vorzugsweise aus 2 bis 4 Kohlenstoffatomen, verzweigtkettige vorzugsweise aus 3 bis 5 Kohlenstoffatomen. Die Hydroxygruppen in den Resten $R^2$ und $R^4$ können als primäre oder sekundäre Hydroxygruppen vorliegen. Die Reste $R^2$ und $R^4$ können 1 bis 5 Hydroxygruppen enthalten, bevorzugt sind 1 bis 3 Hydroxygruppen. Als Reste $R^2$ und $R^4$ seien beispielsweise genannt:
Der 2-Hydroxyethyl-, 2-Hydroxypropyl-, 3-Hydroxypropyl-, 2,3-Dihydroxy-propyl, 1-Hydroxymethyl-2-hydroxy-ethyl-, 2,3-Dihydroxybutyl-, 2,4-Dihydroxybutyl-, 2,4-Dihydroxybutyl-, 3-Hydroxy-2-(hydroxymethyl)-propyl-, 2,3-Dihydroxy-1-methylpropyl-, 2-Hydroxy-3-(hydroxymethyl)-butyl-, 2,3,4-Trihydroxy-butyl-, 2,4-Dihydroxy-3-(hydroxymethyl)-butyl-, 3-Hydroxy-2,2-bis-(hydroxymethyl)-propyl-, 4-Hydroxy-3,3-bis-(hydroxymethyl)-butyl-, 4-Hydroxy-2,2-bis-(hydroxymethyl)-butyl-, 2-Hydroxy-1,1-bis-(hydroxymethyl)-ethyl-, 1,3-Dihydroxy-isopropyl-,2,3-Dihydroxy-1-hydroxy-methyl-propyl-Rest.

Der Rest $R^5$ enthält als niederen Alkylrest vorzugsweise geradkettige Alkylreste mit 1 bis 4 vorzugsweise mit 1 bis 2 Kohlenstoffatomen wie beispielsweise den Ethyl-, Propyl- oder Butyl-, insbesondere den Methylrest.

Stellt $R^5$ einen Mono- oder Polyhydroxyalkylrest dar, so besitzt er 2 bis 6 vorzugsweise 2 bis 4 Kohlenstoffatome und ist mit 1 bis 5 vorzugsweise 1 bis 3 Hydroxygruppen substituiert. Die verwendeten Reste sind beispielsweise 2-Hydroxyethyl, 2,3-Dihydroxypropyl.
X als gerad- oder verzweigtkettiges Alkylen, das durch ein oder mehrere Sauerstoffatome unterbrochen sein kann, kann 1 bis 6 Kohlenstoffatome enthalten. Bevorzugt ist ein geradkettiges Alkylen mit 1 bis 4 Kohlenstoffatomen, das durch ein oder mehrere, vorzugsweise durch ein, zwei oder auch drei Sauerstoffatome unterbrochen sein kann.

Als Beispiele seien hier genannt: $-CH_2-$, $-(CH_2)_4-$, $-(CH_2-CH_2-O-CH_2-CH_2)_2-$ und $-(CH_2-O-CH_2)_3-$.

Als verzweigtkettige Reste X kommen infrage:
> $CHCH_3$, $-[CH_2-C(CH_3)_2-CH_2]-$, $-[CH_2-CH(CH_3)-CH_2CH_2]-$ und ähnliche.

X als gerad- oder verzweigtkettiges Alkylen kann auch durch Hydroxy- oder Alkoxygruppen substituiert sein. So kann jedes Kohlenstoffatom von X eine Hydroxy- oder Alkoxygruppe enthalten.

Als Beispiele seien genannt:
> CHOH, > $CHOCH_3$,

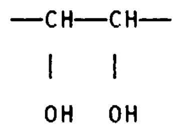

Der Rest X kann auch durch Hydroxyalkyloxygruppen substituiert sein, wie beispielsweise bei

X = > $CH-O-CH_2CH_2-OH$

Röntgenkontrastmittel sind unentbehrliche Hilfsmittel bei der Diagnose zahlreicher Erkrankungen, wie z.B. von arteriosklerotischen Gefäßprozessen, Tumoren, Infarkten, Erkrankungen der Nieren und ableitenden Harnwege. Seit der Einführung der ersten Produkte sind große Fortschritte erzielt worden:

Die chemotoxischen Eigenschaften der Kontrastmittel wurden stark vermindert. Für die klinische Anwendung bedeutet das ein geringeres Auftreten von Nebenwirkungen wie Übelkeit, Erbrechen, von bestimmten Kreislaufreaktionen, Urticaria, Bronchospasmus und anderen Symptomen bis hin zum Schock und Tod. Pharmakologisch sind chemotoxische Effekte z.B. als $LD_{50}$ nach intravenöser Injektion meßbar.

Die früher benutzten Produkte waren sehr stark hyperton (z.B. bis zur 8-fachen Osmolalität des Blutes) und verursachten dementsprechend eine große Zahl von z.T. schwerwiegenden Nebenwirkungen wie z.B. Blutdruckabfall, Bradycardie bis hin zum Herzstillstand, Störungen der Blut-Hirn-Schranke, starke Schmerzen usw.. Neuere Kontrastmittel weisen in den klinisch gebräuchlichen Konzentrationen nur noch die 2- bis 3-fache Osmolalität des Blutes auf.

Obwohl also sowohl die Chemotoxizität als auch die Hypertonizität der Kontrastmittel gesenkt wurden, konnten bis heute keine idealen Werte erreicht werden.

Selbst die neuesten sogenannten nicht-ionischen Kontrastmittel verursachten noch schwere und schwerste Zwischenfälle (McClennan, Radiology 162, 1:1-8 [1987]: "Low-osmolality contrast media: Premises and Promises"), die auf chemisch-toxische Wirkungen zurückgeführt werden müssen.

Auch die Osmolalität dieser Produkte ist noch viel zu hoch, als daß man von physiologischen Kontrastmitteln sprechen könnte. Es ist daher nicht verwunderlich, daß zumindest ein bestimmter Prozentsatz der Patienten über starke Schmerzen bei der Untersuchung mit diesen Produkten klagt ("Pain and hemodynamic effects in aortofemora angiography" in Acta Radiol. Diagnosis 23,4:389-399 [1982]).

Erfahrungsgemäß lassen sich diese Probleme durch die Synthese von wasserlöslichen, sehr hydrophilen "nichtionischen Dimeren" d.h. von Kontrastmittelmolekülen, die aus de Verknüpfung von 2 triiodierten Aromaten bestehen, weitgehend lösen. Solche Substanzen wurden erstmals in der DOS 26 28 517 beschrieben. Seither sind eine Reihe von sehr ähnlichen Strukturen beschrieben worden, z.B. in DOS 28 05 928, EP 0023992, EP 0049745 und EP 0108638.

Nichtionische Dimere sind im allgemeinen bei allen für die Röntgendiagnostik gebräuchlichen Konzentrationen im Vergleich zu den Körperflüssigkeiten nicht hyperton. Darüber hinaus weisen einige Vertreter dieser Substanzklasse eine sehr geringe Chemotoxität auf, d.h. werden extrem hohe $LD_{50}$-Werte nach intravenöser Injektion erzielt.

Trotz dieser Vorzüge haben Kontrastmittel auf Basis nichionischer Dimerer bisher kaum klinische Anwendung gefunden. Die Ursache dafür ist die Viskosität insbesondere der hochkonzentrierten Lösungen, die für bestimmte besonders kritische angiographische Untersuchungen benötigt werden. So sind selektiv angiographische Untersuchungen der Herzkranzgefäße und Ventrikel nur mit Kontrastmittellösungen, die 350 mg Iod/ml oder mehr enthalten, durchzuführen.

Dabei müssen die Kontrastmittellösungen mit sehr hoher Geschwindigkeit durch etwa 100 cm lange sehr enge Katheter injiziert werden. Lösungen mit über 12 bis 15 cP bei 37° C sind dafür kaum mehr geeignet. Aber auch für die sehr rasche intravenöse Injektion, wie sie für verschiedene moderne Röntgentechniken notwendig ist, sind sehr gut verträgliche und wenig visköse Kontrastmittel erforderlich.

Die Viskosität der nicht ionischen dimeren Kontrastmittel ist von einer Reihe von Faktoren abhängig, von denen der Iodgehalt der Moleküle eine wesentliche Rolle spielt. Bei steigendem Iodgehalt nimmt die

Viskosität der Lösungen der betreffenden Moleküle ab, gleichzeitig aber auch ihre Löslichkeit in Wasser.

Aufgabe der vorliegenden Erfindung war es daher, sehr gut verträgliche und wasserlösliche, bei hohen Konzentrationen blutisotone und gleichzeitig wenig visköse Kontrastmittel mit hohem Iodgehalt zur Verfügung zu stellen.

Es war überraschend, daß eine vergleichsweise geringe chemische Veränderung gegenüber dem bekannten Stand der Technik zu den erfindungsgemäßen Verbindungen der Formel I führte, deren wässrige Lösungen neben ausgezeichneter Verträglichkeit und Blutisotonie auch bei Konzentration von 300 bis 400 mg Iod/ml die erwünschte ausreichend neidrige Viskosität besitzen, um eine universelle Anwendung in der Angiographie sowohl bei schneller Zugabe als auch bei der Applikation hoch konzentrierter Lösungen durch enge Katheter zu ermöglichen.

Die erfindungsgemäßen Verbindungen unterscheiden sich strukturell dadurch vom bekannten Stand der Technik, daß die an jedem Benzolkern stehenden Carbamoylgruppen nicht gleich sondern verschieden sind.

Die erfindungsgemäßen Verbindungen der allgemeinen Formel I sind somit als schattengebende Substanzen hervorragend zur Herstellung von bzw. zur Anwendung in Röntgenkontrastmitteln geeignet. Die neuen Verbindungen besitzen alle Eigenschaften, die von Röntgenkontrastmitteln gefordert werden. Viele sind, obwohl nicht-ionisch, sehr gut wasserlöslich. Die neuen Verbindungen stellen hervorragend verträgliche Röntgenkontrastmittel dar, die in der Angiographie, Urographie, Myelographie, Lymphographie und zur Darstellung verschiedener Körperhöhlen und zu anderen radiologischen Untersuchungen geeignet sind.

Aufgrund ihres schwachen und neutralen Geschmackes eignen sich einige der Verbindungen hervorragend für die orale Applikation und zum Einbringen in die Lunge.

Der den gebräuchlichen Kontrastmitteln anhaftende bittere und Übelkeit auslösende Geschmack ist als schwerwiegender Nachteil insbesondere in der Gastrographie und Bronchographie anzusehen.

Die Erfindung betrifft somit auch neue Röntgenkontrastmittel auf Basis von Verbindungen der allgemeinen Formel I. Die Herstellung der neuen Röntgenkontrastmittel auf Basis der erfindungsgemäßen Verbundungen der allgemeinen Formel I erfolgt in an sich bekannter Weise, z.B. dadurch, daß man die schattengebende Substanz mit den in der Galenik üblichen Zusätzen, z.B. Stabilisatoren wie Natriumedetat, Calcium-dinatriumedetat, physiologisch verträglichen Puffern, Natriumchlorid u.ä., in eine für die intravenöse Applikation geeignete Form bringt. Die Konzentration der neuen Röntgenkontrastmittel im wässrigen Medium richtet sich ganz nach der röntgendiagnostischen Methode. Die bevorzugten Konzentrationen und Dosierungen der neuen Verbindungen bewegen sich in den Bereichen von 50-500 mg J/ml für die Konzentration und 5-500 ml für die Dosierung. Besonders bevorzugt sind Konzentrationen zwischen 100-400 mg J/ml.

Die Erfindung betrifft ferner ein Verfahren zur Herstellung von Verbindungen der allgemeinen Formel I, das dadurch gekennzeichnet ist, daß man in an sich bekannter Weise ein Dicarbonsäure-bis[(3-carbamoyl)-(5-chlorcarbonyl)-2,4,6-triiod-anilid)] der allgemeinen Formel II

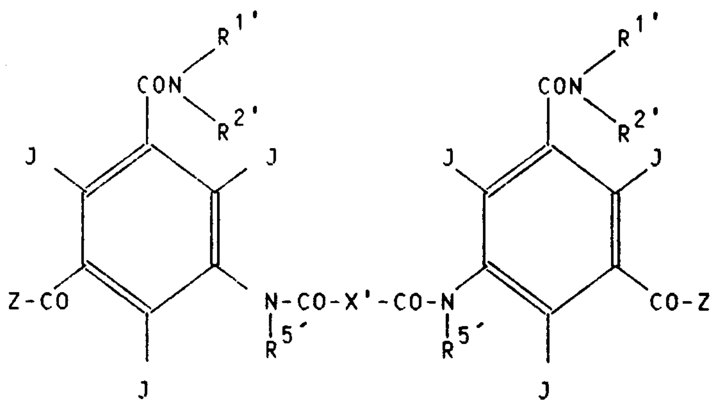

(II)

worin

| | |
|---|---|
| $R^{1'}$ und $R^{2'}$ | die Bedeutung von $R^1$ und $R^2$ besitzen, wobei in $R^1$ und $R^2$ anwesende freie Hydroxylgruppen gegebenenfalls in geschützter Form vorliegen können, |
| X′ | die Bedeutung von X oder eines in X umzuwandelnden Substituenten hat |
| $R^{5'}$ | die Bedeutung eines Wasserstoffatoms oder eines niederen Alkylrestes hat und |
| Z | einen reaktiven Säure- oder Esterrest bedeuten, |

mit einer Base der Formel III

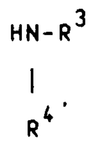

(III)

umsetzt,
worin
$R^{3'}$ und $R^{4'}$ die Bedeutung von $R^3$ und $R^4$ besitzen, wobei in $R^3$ und $R^4$ anwesende freie Hydroxylgruppen gegebenenfalls in geschützter Form vorliegen können und gegebenenfalls anschließend die aromatischen Acylaminogruppen N-alkyliert und zwar im Falle, daß Verbindungen der allgemeinen Formel I mit $R^5$ gleich niederer Alkylrest gewünscht sind, mit einer Verbindung der allgemeinen Formel IV

$R^5$-D (IV)

und im Falle, daß Verbindungen der allgemeinen Formel I mit $R^5$ gleich Mono-oder Polyhydroxylrest gewünscht sind, mit einer Verbindung der allgemeinen Formel V

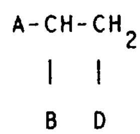

(V)

umsetzt, worin

| | |
|---|---|
| $R^5$ | eine niedere Alkylgruppe, |
| A | ein Wasserstoffatom oder ein Mono- oder Polyhydroxyalkylrest mit 1 bis 4 Kohlenstoffatomen und 1 bis 4 Hydroxylgruppen, darstellt, |
| B und D | entweder gemeinsam einen Oxidoring bilden oder |
| B | eine Hydroxygruppe und |
| D | ein Chlor- oder Bromatom oder eine Sulfat- oder Alkylsulfatgruppe darstellen, |

und im Falle, daß die Gruppe X der Verbindung I eine Methylengruppe bedeutet und alle in der Verbindung I vorhandenen Hydroxylgruppen in geschützter Form vorliegen, gewünschtenfalls mit einem geeigneten hydroxylierenden Agens umsetzt und/oder die Schutzgruppen von geschützten Hydroxygruppen entfernt.

Für die Amidierungsreaktion der Verbindung der Formel II können in den Gruppen $R^{1'}$, $R^{2'}$ und X' anwesende Hydroxylgruppen in freier oder geschützter Form vorliegen. Sollen diese Hydroxylgruppen in geschützter Form vorliegen, kommen alle Hydroxylschutzgruppen infrage, die bekanntermaßen für einen intermediären Hydroxylgruppenschutz geeignet sind, d.h. die sich leicht einführen und sich später unter Rückbildung der letztlich gewünschten freien Hydroxylgruppe auch wieder leicht abspalten lassen. Bevorzugt ist der Schutz durch Veresterung z.B. durch Einführung des Benzoyl- oder Acyl-, insbesondere des Acetylrestes. Geeignete Schutzgruppen sind auch Ethergruppen wie z.B. Benzyl-, Di- und Triphenyl-methyl-Ethergruppen sowie Acetal- und Ketalgruppen mit z.B. Acetaldehyd und Aceton.

Die Amidierung der beiden Carboxylgruppen, die als reaktiver Säure- oder Esterrest Z vorliegen, erfolgt in einem geeigneten Lösungsmittel bei 0° bis 120° C, vorzugsweise bei 20° bis 100° C. Geeignete Lösungsmittel sind u.a. polare Lösungsmittel, wie beispielsweise Wasser, Dioxan, Tetrahydrofuran, Dimethylformamid, Dimethylacetamid, Hexametapol, Aceton u.ä. und deren Gemische. Da bei der Amidierungsreaktion pro umgesetzten Molekül der Verbindung der Formel II zwei Mol Säure (aus dem reaktiven Säure- oder Esterrest) frei werden, die neutralisiert werden müssen, benötigt man für jede reaktive Säure- oder Estergruppe zwei Äquivalente Base, zweckmäßigerweise im Überschuß von mindestens 10 %. Zur praktischen Durchführung wird die gelöste oder suspendierte Ausgangsverbindung der Formel II mit mindestens 4,4 Äquivalenten Base der Formel III oder mit mindestens 2,2 Äquivalenten Base der Formel III und zusätzlich mindestens 2,2 Äquivalenten einer von III verschiedenen Base umgesetzt, die dann als Protonenakzeptor dient. Als Protonenakzeptor verwendet man vorteilhaft tertiäre Amine, wie z.B. Triethylamin, Tributylamin, Pyridin oder Dimethylaminopyridin oder anorganische Basen wie beispielsweise Natriumbicarbonat, Natriumcarbonat oder die entsprechenden Kaliumsalze und deren Hydrate. Die im Reaktionsverlauf

anfallenden anorganischen oder organischen Salze werden in bekannter Weise abgetrennt, z.B. unter Verwendung von Ionentauschern oder durch Filtration über bekannte Adsorbentien, wie z.B. Diaion oder Amberlite® XAD-2 und 4.

Die gegebenenfalls anschließende N-Alkylierung der aromatischen Acylaminogruppen zu Verbindungen der Formel I, worin $R^5$ einen niederen Alkylrest oder einen Mono- oder Polyhydroxyalkylrest bedeutet, erfolgt ebenfalls nach dem Fachmann bekannten Methoden, z.B. in polaren Lösungsmitteln wie Alkanolen oder Alkandiolen, wie Methanol, Ethanol oder Propandiol oder in Polyethern wie Ethylenglykoldiethylether, Diethylenglykoldimethylether u.a. oder deren Gemische in Gegenwart starker Basen, wie der Alkoholate von Natrium, Kalium oder deren Hydride.

Als Alkylierungsmittel dienen im Falle $R^5$ in der Bedeutung niederes Alkyl Verbindungen der allgemeinen Formel IV ($R^5$-D), wie z.B. die Alkyl-halogenide oder -sulfate bzw. deren Äquivalente wie Methyliodid, Methylbromid oder Dimethylsulfat für Verbindungen der Formel I mit $R^5$ = Methyl oder Ethylbromid, Ethyliodid oder Diethylsulfat für Verbindungen der Formel I mit $R^5$ = Ethyl. Zur Herstellung von Vernbindungen der Formel I mit $R^5$ in der Bedeutung eines Mono-oder Polyhydroxyalkylrestes werden Alkylierungsreagentien der allgemeinen Formel V

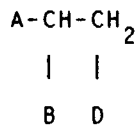

(V)

eingesetzt,
worin
A ein Wasserstoffatom oder eine $CH_2OH$-Gruppe darstellt und
B und D entweder gemeinsam das Sauerstoffatom eines Oxidoringes bedeuten oder
B eine Hydroxygruppe und
D ein Chlor- oder Bromatom oder eine Sulfat- oder Alkylsulfatgruppe darstellen, wie beispielsweise Chlorethanol, Alkylenoxid, Chlorpropandiol-(2,3) oder 2,3-Oxidopropanol bei einer Temperatur von Raumtemperatur bis 80° C vorzugsweise bei 20° C bis 60° C. Alkylierungsreagenz und Alkoholate werden dabei im Überschuß eingesetzt. Zur Aufarbeitung wird nach Abkühlung auf Raumtemperatur in üblicher Weise aufgearbeitet und über Ionenaustauscher entsalzt.

Eine weitere Möglichkeit der Alkylierung besteht darin, die Verbindung I mit $R^5$ in der Bedeutung eines Wasserstoffatomes mit intermediär geschützten Hydroxygruppen in die Reaktion einzusetzen. Dies erfolgt, wie für die Amidierung bereits beschrieben, nach üblichen Methoden durch Einführung von leicht wieder abspaltbaren Gruppen, beispielsweise durch Veretherung (z.B. Einführung des Triphenylmethylrestes).

Der Hydroxylgruppenschutz kann auch durch Ketalisierung oder Acetalisierung, z.B. mittels Acetaldehyd, Aceton, 2,2-Dimethoxypropan oder Dihydropyran, erreicht werden.

Die spätere Abspaltung der intermediär eingeführten Schutzgruppen unter Freisetzung der letztlich gewünschten Hydroxylgruppen erfolgt ebenfalls nach Methoden, wie Sie bereits beschrieben sind (s.o.).
Zur Herstellung der erfindungsgemäßen Verbindungen der Formel I mit X in der Bedeutung von Hydroxymethylen kann man falls erwünscht, auch die Verbindung der Formel I, worin X die Methylengruppe darstellt und alle vorhandenen Hydroxylgruppen in geschützter Form vorliegen mit einem geeigneten Reagenz wie beispeilsweise Bleitetraacetat oder Bleitetrabenzoat hydroxylierend oxydieren, wobei man indifferente Lösungsmittel wie Dioxan, wasserfreie Essigsäure oder Propionsäure verwendet und die Reaktion bei 60° C bis 100° C vorzugsweise bei 80° C bis 100° C durchführt. Anschließend werden - falls erwünscht - die Hydroxyschutzgruppen wieder abgespalten . Es ist auch möglich, einen in X′ enthaltenen Halogen-Substituenten in an sich bekannter Weise in eine Hydroxygruppe umzuwandeln. Z.B. durch Einwirkung von Alkali- oder Silbersalzen niederer Carbonsäuren in polaren Lösungsmitteln bei Temperaturen zwischen 20 und 120°C. Die für die Synthese der Verbindungen der allgemeinen Formel II erforderlichen Zwischenstufen können nach an sich bekannten Verfahren hergestellt werden, beispielsweise aus 5-Nitroisophthalsäure-monoethylester, wie die folgenden Herstellungsvorschriften näher erläutern:

<u>Herstellung von 5-Amino-2,4,6-triiod-isophthalsäure-(2,3-diacetoxy-propyl)-amid-chlorid:</u>

a) 5-Nitro-isophthalsäure-mono-(2,3-dihydroxy-propyl)-amid

239 g (1 mol) 5-Nitro-isophthalsäure-monoethylester und 200,4 g (2,3 mol) Aminopropandiol-2,3 werden 2 Stunden bei ca. 200 mm Quecksilber und 95° C gerührt und das entstehende Ethanol dabei

abdestilliert. Aus der zunächst vorliegenden Suspension entsteht eine klare Schmelze. Die Umsetzung ist nach der angegebenen Zeit vollständig. Die Schmelze wird in 1 Liter Wasser gelöst, die Lösung 30 Minuten bei 60° C mit 24 g Aktivkohle gerührt, filtriert, das Filtrat mit konzentrierter Salzsäure auf pH 1 angesäuert und mit authentischem Material angeimpft. Bei Raumtemperatur kristallisierte das Produkt innerhalb von 15 Stunden reichlich aus. Es wird abgesaugt, mit 250 ml Wasser gewaschen und im Vakuum bei 50° C getrocknet. Die Ausbeute beträgt 260 g (0,914 mol) = 91,4 % der Theorie.

b) 5-Amino-2,4,6-triiod-isophthalsäure-mono-(2,3-dihydroxy-propyl)-amid

284,2 g (1 mol) 5-Nitro-isophthalsäure-mono-(2,3-dihydroxy-propyl)-amid wird in 1 Liter Wasser durch Zufügen von 100 ml 33 %igem wässrigem Ammoniak gelöst, 4 g 20 %iges Palladium-Calziumcarbonat zugegeben und die Lösung bzw. Suspension in einem 5 Liter-Autoklaven innerhalb von einer Stunde zur entsprechenden Amino-Verbindung hydriert. Der Wasserstoffdruck beträgt zu Beginn 40 bar, zum Schluß 6 bar. Die Temperatur steigt zwischenzeitlich auf 45° C. Der Katalysator wird abfiltriert und das Filtrat, in welchem das Zwischenprodukt 5-Amino-isophthalsäure-mono-(2,3-dihydroxy-propyl)-amid gelöst ist, in die Iodierungsreaktion eingesetzt. Dazu wird die Lösung mit 150 ml konzentrierter Salzsäure angesäuert, auf 80° C erwärmt und innerhalb einer Stunde mit 1 Liter (4 mol) 4N $NaICl_2$-Lösung versetzt. Nach beendeter Zugabe wird weitere 3 Stunden bei dieser Temperatur gehalten, die Heizung dann abgeschaltet und 10 Stunden nachgerührt. In dieser Zeit kristallisiert das Produkt aus. Es wird abgesaugt, in 1 Liter Wasser suspendiert, bis zur negativen Reaktion von Kaliumiodid/Stärke-Papier mit $Na_2S_2O_5$ versetzt, das Kristallisat abgesaugt, in 2 Liter Wasser suspendiert, durch Zusatz von 32 %iger Natronlauge gelöst, mit 60 g Aktivkohle 1 Stunde bei 50 bis 60° C gerührt, filtriert, mit konzentrierter Salzsäure angesäuert und erneut kristallisiert. Das Kristallisat wird nach 10 Stunden abgesaugt und bei 50° C im Vakuum getrocknet. Die Ausbeute beträgt 486,6 g (0,77 mol) = 77 % der Theorie bezogen auf die eingesetzte Nitroverbindung.

c) 5-Amino-2,4,6-triiod-isophthalsäure-mono-(2,3-diacetoxy-propyl)-amid

300 g (0,475 mol) 5-Amino-2,4,6-triiod-isophthalsäure-mono-(2,3-dihydroxy-propyl)-amid werden in 1,4 Liter Essigester suspendiert, mit 178,07 g (1,74 mol) Acetanhydrid und 5,7 g (47,5 mmol) 4-Dimethylaminopyridin versetzt und das Gemisch zum Sieden erhitzt. Die Suspension geht in eine Lösung über aus der rasch das Produkt in der Siedehitze auskristallisiert. Die Acetylierung ist nach 1 Stunde beendet. Das überschüssige Acetanhydrid wird durch Zusatz von Ethanol zu Essigester umgesetzt, auf Raumtemperatur gekühlt, das Kristallisat abgesaugt, mit Essigester gewaschen und bei 50° C im Vakuum getrocknet. Die Ausbeute beträgt 300,2 g (0,42 mol) = 88,4 % der Theorie.

d) 5-Amino-2,4,6-triiod-isophthalsäure-(2,3-diacetoxy-propyl)-mono-amid-chlorid

320 g (0,45 mol) 5-Amino-2,4,6-triiod-isophthalsäure-mono-(2,3-diacetoxy-propyl)-amid werden in 1 Liter 1,2-Dichlorethan suspendiert, 107,1 g (0,9 mol) Thionylchlorid zugefügt und auf Siedetemperatur erhitzt. Nach ca. 30 Minuten liegt eine klare Lösung vor, nach 50 Minuten ist die Reaktion vollständig. Es wird auf Raumtemperatur gekühlt, mit 5 Liter 5 %iger Natriumhydrogencarbonat-Lösung 15 Minuten gerührt, die Phasen getrennt, die organische Phase eingedampft. Man erhält 315 g (0,43 mol) = 95,6 % der Theorie amorphes Produkt.

Herstellung von 5-Amino-2,4,6-triiod-isophthalsäure-(2-acetoxy-ethyl)-monoamid-chlorid:

a) 5-Amino-2,4,6-triiod-isophthalsäure-(2-acetoxy-ethyl)-monoamid

128,8 g (200 mmol) 5-Amino-2,4,6-triiod-isophthalsäure-(2-hydroxy-ethyl)-monoamid (DOS 16 43 440) werden in 1,2 Liter Dioxan suspendiert, 61,25 g (600 mmol) Acetanhydrid und 2,44 g (20 mmol) 4-Dimethylaminopyridin hinzugefügt und die Suspension bei 80° C gerührt. Nach ca. 2 Stunden liegt eine annähernd klare Lösung vor und die Umsetzung ist vollständig. Es wird 12 Stunden bei Raumtemperatur gerührt. Das Produkt kristallisiert aus. Es wird abgesaugt, mit Dioxan gewaschen und bei 50° C im Vakuum getrocknet. Die Ausbeute beträgt 122,7 g (190,5 mmol) = 95,3 % der Theorie.

b) 5-Amino-2,4,6-triiod-isophthalsäure-(2-acetoxy-ethyl)-monoamid-chlorid

117,2 g (181,94 mmol) 5-Amino-2,4,6-triiod-isophthalsäure-(2-acetoxy-ethyl)-monoamid werden in 586 ml Dichlorethan suspendiert, 64,94 g (545,82 mmol) Thionylchlorid zugefügt und die Suspension zum Sieden erhitzt. Nach 45 Minuten liegt eine klare Lösung vor, nach 55 Minuten beginnt aus dieser das Sulfinylimid des Produktes kristallin auszufallen. Nach 2 Stunden ist die Umsetzung vollständig. Es wird auf Raumtemperatur gekühlt, das Kristallisat abgesaugt, in 400 ml Dichlorethan suspendiert, dieser Suspension 49,4 g (172,7 mmol) Sodadekahydrat zugefügt und die Suspension 5 Stunden bei Raumtemperatur gerührt. Der Feststoff wird dann abgesaugt und mit 1 Liter Tetrahydrofuran 2 Stunden gekocht. Dabei geht das Produkt in Lösung und anorganische Salze bleiben ungelöst. Die Tetrahydrofuran-Lösung wird auf ca. 500 ml eingeengt, wobei sich das Produkt kristallin abscheidet. Die Ausbeute beträgt

106,73 g (161 mmol) = 88,6 % der Theorie.
Die nachfolgende Dimerisierung zu den Verbindungen der allgemeinen Formel II erfolgt nach literturbekannten Verfahren (z.B. EP 0 033 426)

Beispiel 1

Malonsäure-bis[3-(2,3-dihydroxy-N-methyl-propylcarbamoyl)-5-(2,3-dihydroxy-propylcarbamoyl)-2,4,6-triiod-N-methylanilid]

a) Malonsäure-bis[3-chlorcarbonyl-2,4,6-triiod-5-(2,3-diacetoxy-propylcarbamoyl)-anilid]
79,3 g (108 mmol) 5-Amino-2,4,6-triiod-isophthalsäure-(2,3-diacetoxypropyl)-amid-chlorid werden in 250 ml Toluol gelöst, die Lösung auf 90° erhitzt und 8,81 g (50 mmol) Malonylchlorid hinzugefügt. Es entsteht rasch ein heller kristalliner Niederschlag. Nach 15 Minuten wird die Heizung entfernt und man läßt den Ansatz auf Raumtemperatur kommen. Das kristalline Reaktionsprodukt wird abgesaugt mit Toluol gewaschen und bei 50° C im Vakuum getrocknet. Die Ausbeute beträgt 69,8 g (45,4 mmol) = 84,1 % der Theorie.
b) Malonsäure-bis[3-(2,3-dihydroxy-N-methyl-propylcarbamoyl)-5-(2,3-dihydroxy-propylcarbamoyl)-2,4,6-triiod-anilid]
65 g (42,3 mmol) Malonsäure-bis[3-chlorcarbonyl-2,4,6-triiod-5-(2,3-diacetoxy-propylcarbamoyl)-anilid] werden in 650 ml Aceton gelöst, mit 36,3 g (126,9 mmol) Sodadekahydrat und 13,3 g (126,9 mmol) N-Methyl-aminopropandiol-2,3 versetzt und die Suspension 2 Stunden am Rückfluß gekocht. Das Reaktionsgemisch wird dann auf Raumtemperatur gekühlt, der feste Niederschlag abgesaugt, das Filtrat eingedampft, der Rückstand in 300 ml Wasser gelöst, unter pH-Kontrolle mit konzentrierter Natronlauge verseift, mit Salzsäure neutralisiert und an Ionenaustauschern entsalzt. Das wässrige Eluat vom Ionenaustauscher wird zur Trockne eingedampft. Die Ausbeute beträgt 58,62 g (38,9 mmol) = 92 % der Theorie. Amorpher Feststoff.
c) Malonsäure-bis[3-(2,3-dihydroxy-N-methyl-propylcarbamoyl)-5-(2,3-dihydroxypropylcarbamoyl)-2,4,6-triiod-N-methyl-anilid]
1,48 g (64,3 mmol) Natrium werden in 62 ml Methanol gelöst, die Lösung mit 62 ml Propandiol-1,2 versetzt, in dieser Lösung 23,1 g (15,3 mmol) Malonsäure-bis[3-(2,3-dihydroxy-N-methyl-propylcarbamoyl)-5-(2,3-dihydroxy-propylcarbamoyl)-2,4,6-triiod-anilid] gelöst und 4 Stunden bei 50° C gerührt. Das Methanol wird dann bei Normaldruck abdestilliert, 7,72 g (61,2 mmol) Dimethylsulfat zugefügt und 20 Stunden bei 50° C gerührt. Die Reaktionslösung wird dann auf Raumtemperatur gekühlt, in 1 Liter Aceton gefällt, die Fällung abgesaugt, in Wasser gelöst und an Ionenaustauscher entsalzt. Das Eluat wird zum amorphen Feststoff eingedampft. Die Ausbeute beträgt 21,12 g (13,8 mmol) = 90 % der Theorie.

Beispiel 2

Malonsäure-bis{3-(2,3-dihydroxy-propylcarbamoyl)-5-[bis(2-hydroxyethyl)-carbamoyl]-2,4,6-triiod-N-methyl-anilid}

a) Malonsäure-bis{3-(2,3-dihydroxy--propylcarbamoyl)-5-[bis(2-hydroxyethyl)-carbamoyl]-2,4,6-triiod-anilid}
153,7 g (100 mmol) Malonsäure-bis[3-chlorcarbonyl-2,4,6-triiod-5-(2,3-diacetoxy-propylcarbamoyl)-anilid] werden bei Raumtemperatur in 1537 ml Aceton gelöst, die Lösung mit 85,84 g (300 mmol) Sodadekahydrat versetzt und zu dieser Suspension im Verlaufe von ca. 15 Minuten eine Lösung von 31,54 g (300 ml) Diethanolamin in 100 ml Aceton zugetropft. Die Suspension wird dann 1,5 Stunden am Rückfluß gekocht. Nach Ablauf dieser Zeit ist die Umsetzung vollständig. Der feste Bodenkörper wird abgesaugt, das Filtrat zu einem Öl eingeengt, dieses Öl in 300 ml Wasser gelöst und bei 50° C mit 32 %iger Natronlauge so lange bei pH 12 gehalten, bis der pH-Wert bei 12 bleibt und die Dünnschichtchromatographie vollständige Verseifung der Acetatgruppen anzeigt. Die wässrige Lösung wird mit wässriger Salzsäure neutralisiert und an Ionenaustauschern entsalzt. Das wässrige Eluat wird zur Trockne eingedampft. Die Ausbeute beträgt 130 g (86,3 mmol) = 86,3 % der Theorie als farbloser amorpher Feststoff.
b) Malonsäure-bis{3-(2,3-dihydroxy-propylcarbamoyl)-5-[bis(2-hydroxyethyl)-carbamoyl]-2,4,6-triiod-N-methyl-anilid}
4,6 g (200 mmol) Natrium werden in einem Gemisch aus 200 ml Methanol und 200 ml Propandiol-1,2 gelöst, dieser Lösung 75,3 g (50 mmol) Malonsäure-bis{3-(2,3-dihydroxy-propylcarbamoyl)-5-[bis(2-hydroxyethyl)-carbamoyl]-2,4,6-triiod-anilid} zugefügt, 3 Stunden bei 50° C gerührt und das Methanol

dann bei Normaldruck abdestilliert. Die Reaktionslösung wird darauf mit 28,67 g (200 mmol) Methyliodid versetzt und 24 Stunden bei 50° C gerührt. Die Dünnschichtchromatographie zeigt dann vollständige Umsetzung an. Die Reaktionslösung wird auf Raumtemperatur gekühlt, in 2 Liter Aceton eingerührt, die amorphe Fällung des Produktes abgesaugt, in Wasser gelöst und die Lösung an Ionenaustauschern entsalzt. Das zur Trockne eingedampfte wässrige Eluat ergibt 60,2 g (39,25 mmol) = 78,5 % der Theorie eines farblosen amorphen Feststoffes.

Beispiel 3

Malonsäure-bis{3-(2,3-dihydroxy-propylcarbamoyl)-5-[(1RS,2SR)-2,3-dihydroxy-1-hydroxymethylpropyl-carbamoyl]-2,4,6-triiod-N-methyl-anilid}

a) Malonsäure-bis{3-(2,3-dihydroxy-propylcarbamoyl)-5-[(1RS,2SR)-2,3-dihydroxy-1-hydroxymethylpropyl-carbamoyl]-2,4,6-triiod-anilid}
153,7 g (100 mmol) Malonsäure-bis{3-chlorcarbonyl-2,4,6-triiod-5-(2,3-diacetoxy-propylcarbamoyl)-anilid] werden in 1,52 Liter Aceton bei Raumtemperatur gelöst, 85,84 g (300 mmol) Sodadekahydrat und 48,36 g (300 mmol) 6-Amino-2,2-dimethyl-1,3-dioxepin-5-ol hinzugefügt und 2 Stunden am Rückfluß gekocht. Das Reaktionsgemisch wird dann auf Raumtemperatur gekühlt, der feste Niederschlag abgesaugt, das Filtrat eingedampft, der Rückstand in 300 ml Wasser gelöst und unter pH-Kontrolle bei 50° C die Acetatgruppen bei pH 12 und die Ketale bei pH 1 hydrolysiert. Anschließend wird die Lösung neutral gestellt und an Ionenaustauschern entsalzt. Das wässrige Eluat wird zur Trockne eingedampft und ergibt 134,28 g (87,3 mmol) = 87,3 % der Theorie amorphen Feststoff.

b) Malonsäure-bis{3-(2,3-dihydroxy-propylcarbamoyl)-5-[(1RS,2SR)-2,3-dihydroxy-1-hydroxymethylpropyl-carbamoyl]-2,4,6-triiod-N-methyl-anilid}
9,2 g (400 mmol) Natrium werden in einem Gemisch aus 400 ml Methanol und 400 ml Propandiol-1,2 gelöst, dieser Lösung 153,8 g (100 mmol) Malonsäure-bis{3-(2,3-dihydroxy-propylcarbamoyl)-5-[-(1RS,2SR)-2,3-dihydroxy-1-hydroxymethylpropyl-carbamoyl]-2,4,6-triiod-anilid} zugefügt, 3 Stunden bei 50° C gerührt und das Methanol dann bei Normaldruck abdestilliert. Die Reaktionslösung wird darauf mit 57,35 g (400 mmol) Methyliodid versetzt und 24 Stunden bei 50° C gerührt. Die DC zeigt dann vollständige Umsetzung. Die Reaktionslösung wird auf Raumtemperatur gekühlt, in 4 Liter Aceton eingerührt, die amorphe Fällung des Produktes abgesaugt, in Wasser gelöst und die wässrige Lösung an Ionenaustauschern entsalzt. Das zur Trockne eingedampfte Eluat ergibt 116,84 g (74,6 mmol) = 74,6 % der Theorie eines farblosen amorphen Feststoffes.

Beispiel 4

Malonsäure-bis[3-(2-hydroxy-N-methyl-ethyl-carbamoyl)-5-(2,3-dihydroxy-propylcarbamoyl)-2,4,6-triiod-N-(2-hydroxy-ethyl)-anilid]

a) Malonsäure-bis[3-(2-hydroxy-N-methyl-ethyl-carbamoyl)-5-(2,3-dihydroxy-propyl-carbamoyl]-2,4,6-triiod-anilid]
153,7 g (100 mmol) Malonsäure-bis[3-chlorcarbonyl-5-(2,3-diacetoxy-propylcarbamoyl)-2,4,6-triiod-anilid] werden in 1,52 Liter Aceton bei Raumtemperatur gelöst, 85,84 g (300 mmol) Sodadekahydrat und 22,53 g (300 mmol) N-Methyl-ethanolamin hinzugefügt und 2 Stunden am Rückfluß gekocht, Die Suspension wird dann auf Raumtemperatur gekühlt, der feste Niederschlag abgesaugt, das Filtrat eingedampft, der Rückstand in 300 ml Wasser gelöst und unter pH-Kontrolle bei 50° C die Acetatgruppen bei pH 12 verseift. Anschließend wird die Lösung neutralisiert und an Ionenaustauschern entsalzt. Das wässrige Eluat wird zur Trockne eingedampft und ergibt 109,1 g (75,5 mmol) = 75,5 % der Theorie eines farblosen amorphen Feststoffes.

b) Malonsäure-bis[3-(2-hydroxy-N-methyl-ethyl-carbamoyl)-5-(2,3-dihydroxy-propylcarbamoyl)-2,4,6-triiod-N-(2-hydroxy-ethyl)-anilid]
4,29 g (186,76 mmol) Natrium werden in einem Gemisch von 193 ml Methanol und 193 ml Propandiol-1,2 gelöst, 64,3 g (44,46 mmol) Malonsäure-bis[3-(2-hydroxy-N-methyl-ethyl-carbamoyl)-5-(2,3-dihydroxy-propylcarbamoyl)-2,4,6-triiod-anilid] hinzufügt, die Lösung 3 Stunden bei 50° C gerührt und das Methanol dann bei Normaldruck abdestilliert. Die Reaktionslösung wird darauf mit 14,61 g (177,84 mmol) Chlorethanol versetzt und 24 Stunden bei 50° C gerührt. Die Dünnschichtchromatographie zeigt dann vollständige Umsetzung. Die Reaktionslösung wird auf Raumtemperatur gekühlt, in 3 Liter Aceton eingerührt, die amorphe Fällung des Produktes abgesaugt, in Wasser gelöst und die wässrige Lösung in

Ionenaustauschern entsalzt. Das zur Trockne eingedampfte Eluat ergibt 51,57 g (33,68 mmol) = 75,7 % der Theorie eines farblosen amorphen Feststoffes.

Beispiel 5

Malonsäure-bis[3-(2,3-dihydroxy-N-methyl-propyl-carbamoyl)-5-(2-hydroxy-ethylcarbamoyl]-2,4,6-triiod-N-methyl-anilid]

a) Malonsäure-bis[3-chlorcarbonyl-2,4,6-triiod-(2-acetoxy-ethylcarbamoyl)-anilid
155,9 g (235.4 mmol) 5-Amino-2,4,6-triiod-isophthalsäure-(2-acetoxy-ethyl)-monoamid-chlorid werden bei Raumtemperatur in 780 ml Dioxan suspendiert, auf 80° C erhitzt, bis eine Lösung entstanden ist. Dazu werden 20,32 g (141,2 mmol) Malonylchlorid zugefügt und 12 Stunden bei 80° C gerührt. Das Produkt kristallisiert in dieser Zeit zunehmend aus der Reaktionslösung aus. Es wird auf Raumtemperatur gekühlt, das Kristallisat abgesaugt, mit Dioxan gewaschen und bei 50° C im Vakuum 24 Stunden getrocknet. Die Ausbeute beträgt 123,26 g (88,5 mmol) = 75,2 % der Theorie.
b) Malonsäure-bis[3-(2,3-dihydroxy-N-methyl propyl-carbamoyl)-5-(2-hydroxy-ethylcarbamoyl]-2,4,6-triiod-anilid]
69,64 g (50 mmol) Malonsäure-bis[3-chlorcarbonyl-2,4,6-triiod-(2-acetoxyethylcarbamoyl)-anilid] werden in 105 ml Dimethylformamid gelöst und bei Raumtemperatur zu dieser Lösung das in 10 ml DMF gelöste Gemisch aus 15,78 g (150 mmol) N-Methyl-aminopropandiol-1,2 und 15,18 g (150 mmol) Triethylamin zugetropft. Nach vierstündigem Rühren bei Raumtemperatur ist die Umsetzung vollständig. Das Hydrochlorid des Triethylamins wird abfiltriert, das Filtrat in 2 Liter Methylenchlorid eingerührt, der Niederschlag des Produktes abgesaugt, in 200 ml Wasser gelöst, diese Lösung bei 50° C und pH 12 verseift, mit Salzsäure neutralisiert und in Ionenaustauschern entsalzt. Das zur Trockne eingedampfte Eluat ergibt 56,1 g (38,8 mmol) = 77,6 % der Theorie farblosen amorphen Feststoff.
c) Malonsäure-bis[3-(2,3-dihydroxy-N-methyl-propyl-carbamoyl)-5-(2-hydroxy-ethylcarbamoyl)-2,4,6-triiod-N-methyl-anilid]
4,6 g (200 mmol) Natrium werden in einem Gemisch von 200 ml Methanol und 200 ml Propandiol-1,2 gelöst, der Lösung 72,3 g (50 mmol) Malonsäure-bis[3-(2,3-dihydroxy-N-methyl-propyl-carbamoyl)-5-(2-Hydroxy-ethylcarbamoyl)-2,4, 6-triiod-anilid] zugefügt, die Lösung 3 Stunden bei 50° C gerührt, das Methanol dann bei Normaldruck abdestilliert, 28,4 g (200 mmol) Methyliodid zugefügt und 24 Stunden bei 50° C gerührt. Gemäß Dünnschichtchromatographie ist die Umsetzung nach Ablauf dieser Zeit vollständig. Die Reaktionslösung wird auf Raumtemperatur gekühlt, in 3 Liter Aceton eingerührt, die Fällung des Produktes abgesaugt, in Wasser gelöst und die wässrige Lösung an Ionenaustauschern entsalzt. Das zur Trockne eingedampfte Eluat enthält 60,06 g (39,15 mmol) = 78,3 % der Theorie eines farblosen amorphen Feststoffes.

Beispiel 6

Malonsäure-bis{3-(2,3-dihydroxy-propylcarbamoyl)-5-(2-hydroxyethyl-carbamoyl)-2,4,6-triiod-N-(2,3-dihydroxy-propyl)-anilid}

a) Malonsäure-bis{3-(2,3-dihydroxy-propylcarbamoyl)-5-(2-hydroxyethyl-carbamoyl]-2,4,6-triiod-anilid}
100 g (68,45 mmol) Malonsäure-bis[3-chlorcarbonyl-2,4,6-triiod-5-(2,3-diacetoxypropylcarbamoyl)-anilid] werden in 1000 ml Aceton gelöst, die Lösung mit 59,54 g (208,1 mmol) Sodadekahydrat versetzt und zu dieser Suspension im Verlaufe von ca. 15 Minuten 11,29 g (184,82 mmol) Ethanolamin, gelöst in 50 ml Aceton, zugetropft. Nach beendeter Zugabe des Amins wird die Suspension 2 Stunden am Rückfluß gekocht. Gemäß Dünnschichtchromatographie war die Umsetzung dann vollständig, das Produkt befindet sich im Niederschlag der anorganischen Salze. Der Niederschlag wird abgesaugt, in Wasser suspendiert, das in Wasser schwer lösliche Zwischenprodukt abgesaugt, der Filterrückstand in Wasser suspendiert, mit Natronlauge verseift, neutralisiert, die wässrige Lösung in Ionenaustauschern entsalzt und das Eluat zur Trockne eingedampft. Ausbeute 84,73 g (59,76 mmol) = 87,3 % der Theorie als farbloser amorpher Feststoff.
b) Malonsäure-bis{3-(2,3-dihydroxy-propylcarbamoyl)-5-(2-hydroxyethyl-carbamoyl)-2,4,6-triiod-N-(2,3-dihydroxy-propyl)-anilid}
9,2 g (400 mmol) Natrium werden in einem Gemisch aus 400 ml Methanol und 400 ml Propandiol-1,2 gelöst, dieser Lösung 141,8 g (100 mmol) Malonsäure-bis{3-(2,3-dihydroxy-propylcarbamoyl)-5-(2-hydroxyethyl-carbamoyl)-2,4,6-triiod-anilid} zugefügt, 3 Stunden bei 50° C gerührt und das Methanol dann

bei Normaldruck abdestilliert. Die Reaktionslösung wird mit 32,86 g (300 mmol) Chlorpropandiol-2,3 versetzt und 24 Stunden bei 50° gerührt. Die Umsetzung ist gemäß Dünnschichtchromatographie vollständig. Zur Aufarbeitung wird die Reaktionslösung in 4 Liter Aceton eingerührt, die Fällung des Produktes abgesaugt, in Wasser gelöst, an Ionenaustauschern entsalzt, das wässrige Eluat zur Trockne eingedampft. Es ergibt sich 127,2 g (81,2 mmol) = 81,2 % der Theorie des Produktes als farbloser amorpher Feststoff.

Beispiel 7

Hydroxymalonsäure-bis[3-(2,3-dihydroxy-propylcarbamoyl)-5-(2-hydroxyethyl-carbamoyl)-2,4,6-triiod-N-(2,3-dihydroxy-propyl)-anilid]

a) Acetoxymalonsäure-bis[3-chlorcarbonyl-2,4,6-triiod-5-(2,3-diacetoxy-propylcarbamoyl)-anilid]
73,4 g (100 mmol) 5-Amino-2,4,6-triiod-isophthalsäure-(2,3-diacetoxypropyl)-amid-chlorid werden in 250 ml Toluol gelöst, die Lösung auf 90° C erwärmt und 10,12 g (55 mmol) Acetoxymalonsäuredichlorid (hergestellt in Analogie zu O-Acetyl-lactylchlorid, Filachione et al. JACS 72, 410 [1950]) eingetropft. Es entsteht rasch ein kristalliner Niederschlag des Produktes. Nach 15 Minuten wird das Heizbad entfernt, auf Raumtemperatur gekühlt, das Kristallisat abgesaugt, mit Toluol gewaschen und bei 50° im Vakuum getrocknet. Die Ausbeute beträgt 55,97 g (36,9 mmol) = 73,8 % der Theorie.
b) Hydroxymalonsäure-bis[3-(2,3-dihydroxy-propylcarbamoyl)-5-(2-hydroxyethyl-carbamoyl]-2,4,6-triiod-anilid]
84,95 g (56 mmol) Acetoxymalonsäure-bis[3-(2,3-diacetoxy-propylcarbamoyl)-5-chlorcarbonyl-2,4,6-triiod-anilid] werden in 170 ml Dioxan gelöst, 41,66 g (145,6 mmol) Sodadekahydrat und 8,9 g (145,6 mmol) Ethanolamin zugefügt und 2 Stunden am Rückfluß gekocht. Das Reaktionsgemisch wird dann auf Raumtemperatur gekühlt, der Niederschlag abgesaugt, das Filtrat eingedampft, der Rückstand in 250 ml Wasser gelöst, unter pH-Kontrolle bei 50° C mit konzentrierter Natronlauge verseift, mit Salzsäure neutralisiert und an Ionenaustauschern entsalzt. Das wässrige Eluat wird zur Trockne eingedampft. Die Ausbeute beträgt 71 g (49,5 mmol) = 88,4 % der Theorie.
c) Hydroxymalonsäure-bis[3-(2,3-dihydroxy-propyl-carbamoyl)-5-(2-hydroxyethyl-carbamoyl]-2,4,6-triiod-N-(2,3-dihydroxy-propyl)-anilid]
4,6 g (200 mmol) Natrium werden in einem Gemisch aus 200 ml Methanol und 200 ml Propandiol-1,2 gelöst, dieser Lösung 71,7 g (50 mmol) Hydroxymalonsäure-bis[3-(2,3-dihydroxy-propyl-carbamoyl)-5-(2-hydroxyethyl-carbamoyl)-2,4,6-triiod-anilid] zugefügt, 3 Stunden bei 50° C gerührt und das Methanol dann bei Normaldruck abdestilliert. Die Reaktionslösung wird anschließend mit 16,43 g (150 mmol) Chlorpropandiol-2,3 versetzt und 24 Stunden bei 50° C gerührt. Zur Aufarbeitung wird in 3 Liter Methylenchlorid eingerührt, die Fällung abgesaugt, in 200 ml Wasser gelöst, an Ionenaustauschern entsalzt, das Eluat zur Trockne eingedampft und der Rückstand bei 50° C im Vakuum getrocknet. Die Ausbeute beträgt 60,91 g (38,5 mmol) = 77 % der Theorie.

Beispiel 8

Hydroxymalonsäure-bis[3-(2-hydroxy-1-hydroxymethylethyl-carbamoyl)-5-(2,3-dihydroxy-propyl-carbamoyl)-2,4,6-triiod-N-methyl-anilid]

a) Hydroxymalonsäure-bis[3-(2-hydroxy-1-hydroxymethylethyl-carbamoyl)-5-(2,3-dihydroxy-propyl-carbamoyl)-2,4,6-triiod-anilid]
75,85 g (50 mmol) Acetoxymalonsäure-bis[3-(2,3-diacetoxy-propyl-carbamoyl)-5-chlorcarbonyl-2,4,6-triiod-anilid]werden in 150 ml Dioxan gelöst, 37,2 g (130 mmol) Sodadekahydrat und 11,84 g (130 mmol) Serinol zugefügt und 2 Stunden am Rückfluß gekocht. Das Reaktionsgemisch wird dann auf Raumtemperatur gekühlt, der Feststoff abgesaugt, das Filtrat eingedampft, der Rückstand in 250 ml Wasser gelöst, unter pH-Kontrolle bei 50 °C mit konzentrierter Natronlauge verseift, mit Salzsäure neutralisiert und an Ionenaustauschern entsalzt. Das zur Trockne eingedampfte wässrige Eluat enthält 64,62 g (43,25 mmol) = 86,5 % der Theorie farblosen Feststoff.
b) Acetoxymalonsaure-bis[3-(2-acetoxy-1-acetoxymethylethyl-carbamoyl)-5-(2,3-diacetoxy-propyl-carbamoyl)-2,4,6-triiod-anilid]
112,06 g (75 mol) Hydroxymalonsäure-bis [3-(2-hydroxy-1-hydroxymethylethyl-carbamoyl-)5-(2,3-dihydroxy-propyl-carbamoyl)-2,4,6-triiod-anilid] werden in 300 ml Essigester suspendiert, 137,8 g (1,35 mmol) Acetanhydrid und 1,65 g (13,5 mmol) 4-Dimethylamino-pyridin zugefügt und 5 Stunden am

Rückfluß gekocht. Es liegt dann eine klare Lösung vor und die Umsetzung ist quantitativ. Diese Lösung wird mit 31,1 g (0,675 mmol) Ethanol versetzt und eine weitere Stunde am Rückfluß gekocht. Dann wird das Lösungsmittel weitgehend abdestilliert, der Rückstand mit 300 ml Wasser verrührt, das dabei fest anfallende Produkt abgesaugt und getrocknet. Ausbeute 115,6 g (61,7 mmol) = 82,3 % der Theorie.

c) Hydroxymalonsäure-bis[3-(2-hydroxy-1-hydroxymethylethyl-carbamoyl)-5-(2,3-dihydroxy-propyl-carbamoyl)-2,4,6-triiod-N-methyl-anilid]

4,6 g (200 mmol) Natrium werden in einem Gemisch aus 200 ml Methanol und 200 ml Ethylenglykoldimethylether gelöst, 93,62 g (50 mmol) Acetoxymalonsäure-bis [3-(2-acetoxy-1-acetoxymethylethyl-carbamoyl)-5(2,3-diacetoxy-propyl-carbamoyl)-2,4,6-triiod-anilid] zugefügt, 3 Stunden bei 50° C gerührt, das Methanol dann bei Normaldruck abdestilliert, der verbleibenden Lösung 21,3 g (150 mmol) Methyliodid zugefügt und 24 Stunden bei 50° C gerührt. Die Reaktionslösung wird dann filtriert, das Filtrat eingedampft, der Rückstand in Wasser suspendiert, mit Natronlauge verseift, neutralisiert, die Lösung an Ionenaustauschern entsalzt und das Eluat zur Trockne eingedampft. Man erhält 55,25 g (36,3 mmol) = 72,6 % der Theorie farblosen Feststoff.

## Beispiel 9

Hydroxymalonsäure-bis[3-(2-hydroxy-N-methyl-ethylcarbamoyl)-5-(2,3-dihydroxypropyl-carbamoyl)-2,4,6-triiod-N-(2-hydroxy-ethyl)-anilid]

a) Malonsäure-bis[3-(2-acetoxy-N-methyl-ethylcarbamoyl)-5-(2,3-diacetoxy-propylcarbamoyl)-2,4,6-triiod-N-(2-acetoxy-ethyl)-anilid]

76,78 g (50 mmol) Malonsäure-bis[3-(2-hydroxy-N-methyl-ethylcarbamoyl)-5-(2,3-dihydroxy-propylcarbamoyl)-2,4,6-triiod-N-(2-hydroxy-ethyl)-anilid] (siehe Beispiel 4) werden in 400 ml Dioxan suspendiert, 122,5 g (1,2 mol) Acetanhydrid und 0,61 g (5 mmol) 4-Dimethylaminopyridin zugefügt und das Gemisch auf 80° C erwärmt. Nach ca. 1 Stunde liegt eine klare Lösung vor, nach 2 Stunden ist die Acetylierung vollständig. Das überschüssige Acetanhydrid wird durch Zugabe von 36,85 g (800 mmol) Ethanol in Essigester überführt, die Lösung unter vermindertem Druck aufkonzentriert und das Konzentrat in 2 Liter Wasser eingerührt. Die sich dabei ergebende flockige Fällung wird abgesaugt, mit Wasser gewaschen und im Vakuum bei 50° C getrocknet. Die Ausbeute betragt 80.8 g (43,2 mmol) = 86,4 % der Theorie.

b) Hydroxymalonsäure-bis[3-(2-hydroxy-N-methyl-ethylcarbamoyl)-5-(2,3-dihydroxy-propylcarbamoyl)-2,4,6-triiod-N-(2-hydroxy-ethyl)-anilid]

74,8 g (40 mmol) Malonsäure-bis[3-(2-acetoxy-N-methyl-ethylcarbamoyl)-5-(2,3-diacetoxy-propylcarbamoyl)-2,4,6-triiod-N-(2-acetoxy-ethyl)-anilid] werden in 250 ml Essigsäure gelöst, die Lösung auf 100° C erwärmt und in mehreren Portionen innerhalb von ca. 45 Minuten 17,73 g (40 mmol) Bleitetraacetat hinzugefügt. Nach beendeter Zugabe wird weitere 2 Stunden bei 100° C gehalten. Ein Teil der Essigsäure wird dann bei vermindertem Druck abdestilliert, die aufkonzentrierte Lösung auf Raumtemperatur gekühlt und in 3 Liter Wasser eingerührt. Das Produkt fällt dabei als flockiger Niederschlag aus. Dieser wird abgesaugt, mit Wasser gewaschen und wasserfeucht in 300 ml Ethanol gelöst. Zu dieser Lösung fügt man bei 50° C so lange 32 %ige Natronlauge, bis die Dünnschichchromatographie die vollständige Hydrolyse der Acetylgruppen anzeigt. Die Lösung wird dann neutralisiert, das Ethanol weitgehend bei vermindertem Druck abdestilliert, durch 200 ml Wasser ersetzt und diese Lösung an Ionenaustauschern entsalzt. Durch Eindampfen des wässrigen Eluats erhält man 38,13 g (24,6 mmol) = 61,5 % der Theorie Produkt.

## Beispiel 10

Methoxymalonsäure-bis[3-(2-hydroxy-1-hydroxymethylethyl-carbamoyl)-5-(2,3-dihydroxy-propylcarbamoyl)-2,4,6-triiod-N-methyl-anilid]

a) Methoxymalonsäure-bis[3-chlorcarbonyl-2,4,6-triiod-5-(2,3-diacetoxy-propylcarbamoyl)-anilid]

73,4 g (100 mmol) 5-Amino-2,4,6-triiod-isophthalsäure-(2,3-diacetoxypropyl)-amid-chlorid wird in 250 ml Toluol gelöst, die Lösung auf 90° C erwärmt und 8,52 g (55 mmol) Methoxymalonylchlorid eingetropft. Es entsteht rasch ein kristalliner Niederschlag des Produktes. Nach 15 Minuten wird das Heizbad entfernt, auf Raumtemperatur gekühlt, das Kristallisat abgesaugt, mit Toluol gewaschen und bei 50° C im Vakuum getrocknet. Die Ausbeute beträgt 57,07 g (38,15 mmol) = 76,3 % der Theorie.

b) Methoxymalonsäure-bis[3-(2-hydroxy-1-hydroxymethylethyl-carbamoyl)-5-(2,3-dihydroxy-propylcarbamoyl)-2,4,6-triiod-anilid]

74,8 g (50 mmol) Methoxymalonsäure-bis[3-chlorcarbonyl-5-(2,3-diacetoxy-propylcarbamoyl)-2,4,6-triiod-anilid] werden in 150 ml Dioxan gelöst, 37,2 g (130 mmol) Sodadekahydrat und 11,84 g (130 mmol) Serinol zugefügt und 2 Stunden bei 50° C gerührt. Das Reaktionsgemisch wird dann auf Raumtemperatur gekühlt, der Feststoff abgesaugt, das Filtrat eingedampft, der Rückstand in 250 ml Wasser gelöst, unter pH-Kontrolle bei 50° C mit konzentrierter Natronlauge verseift, mit Salzsäure neutralisiert und an Ionenaustauschern entsalzt. Das zur Trockne eingedampfte wässrige Eluat enthält 66,58 g (44,15 mmol) = 88,3 % der Theorie farblosen amorphen Feststoff.

c) Methoxymalonsäure-bis[3-(2-hydroxy-1-hydroxymethylethyl-carbamoyl)-5-(2,3-dihydroxy-propylcarbamoyl)-2,4,6-triiod-N-methyl-anilid]

4,6 g (200 mmol) Natrium werden in einem Gemisch aus 200 ml Methanol und 200 ml Propandiol-1,2 gelöst, 75,4 g (50 mmol) Methoxymalonsäure-bis[3-(2-hydroxy-1-hydroxymethylethyl-carbamoyl)-5-(2,3-dihydroxy-propylcarbamoyl)-2, 4,6-triiod-anilid] zugefügt, 3 Stunden bei 50° C gerührt, das Methanol dann bei Normaldruck abdestilliert, der verbleibenden Lösung 21,3 g (150 mmol) Methyliodid zugefügt und 24 Stunden bei 50° C gerührt. Die Reaktionslösung wird dann auf Raumtemperatur gekühlt und in 2 Liter Methylenchlorid eingerührt. Das Produkt fällt dabei als pastöse Masse aus. Von dieser wird dekantiert, in 200 ml Wasser gelöst und an Ionenaustauschern entsalzt. Man erhielt 56,4 g (36,7 mmol) = 73,4 % der Theorie der Titelverbindung als amorphen Feststoff.

Beispiel 11

2,3-Dihydroxy-bernsteinsäure-bis[3-(2,3-dihydroxy-propylcarbamoyl)-5-(2-hydroxy-ethylcarbamoyl)-2,4,6-triiod-N-(2-hydroxyethyl)-anilid]

a) 2,3-Diacetoxy-bernsteinsäure-bis[3-chlorcarbonyl-2,4,6-triiod-5-(2,3-diacetoxy-propylcarbamoyl)-anilid]

73,4 g (100 mmol) 5-Amino-2,4,6-triiod-isophthalsäure-(2,3-diacetoxy-propyl)-amid-chlorid werden in 250 ml Toluol gelöst, die Lösung auf 90° C erwärmt und 14,91 g (55 mmol) 2,3-Diacetyl-bernsteinsäuredichlorid (Herstellung nach D. Seebach et al. Ber. 1980, 1691) hinzugefügt. Es entsteht rasch ein kristalliner Niederschlag des Produktes. Nach 30 Minuten wird auf Raumtemperatur gekühlt und das Kristallisat abgesaugt, mit Toluol gewaschen und bei 50° C im Vakuum getrocknet. Die Ausbeute beträgt 59,43 g (35,65 mmol) = 71,3 % der Theorie.

b) 2,3-Dihydroxy-bernsteinsäure-bis[3-(2,3-dihydroxy-propylcarbamoyl)-5-(2-hydroxy-ethylcarbamoyl)-2,4,6-triiod-anilid]

75,02 g (45 mmol) 2,3-Diacetoxy-bernsteinsäure-bis[3-(2,3-diacetoxypropylcarbamoyl)-5-chlorcarbonyl-2,4,6-triiod-anilid] werden in 250 ml Dioxan gelöst, 15,7 g (67,5 mmol) Sodadekahydrat und 4,12 g (67,5 mmol) Ethanolamin hinzugefügt und 2 Stunden bei 50° C gerührt. Die Suspension wird dann auf Raumtemperatur gekühlt, der Niederschlag abgesaugt, das Filtrat eingedampft, der Rückstand in 250 ml Wasser suspendiert, bei 50° C unter pH-Kontrolle mit konzentrierter Natronlauge verseift, mit Salzsäure neutralisiert und an Ionentauschern entsalzt. Das wässrige Eluat wird zur Trockne eingedampft und ergibt 57,65 g (39,4 mmol) 87,6 % der Theorie der Verbindung.

c) 2,3-Dihydroxy-bernsteinsäure-bis[3-(2,3-dihydroxy-propylcarbamoyl)-5-(2-hydroxy-ethylcarbamoyl)-2,4,6-triiod-N-(2-hydroxy-ethyl)-anilid]

4,6 g (200 mmol) Natrium werden in einem Gemisch aus 200 ml Methanol und 200 ml Propandiol-2,3 gelöst, dieser Lösung werden 73,2 g (50 mmol) 2,3-Dihydroxy-bernsteinsäure-bis[3-(2,3-dihydroxy-propylcarbamoyl)-5-(2-hydroxy-ethylcarbamoyl)-2,4,6-triiod-anilid] zugefügt, 3 Stunden bei 50° C gerührt und das Methanol dann bei Normaldruck abdestilliert. Die Reaktionslösung wird anschließend mit 12,1 g (150 mmol) Chlorethanol versetzt und 24 Stunden bei 50° C gerührt. Zur Isolierung des Produktes wird die Reaktionslösung nach Abkühlen auf Raumtemperatur in 3 Liter Methylenchlorid gefällt, die Fällung abgesaugt, in Wasser gelöst und an Ionenaustauschern entsalzt. Das zur Trockne eingedampfte Eluat ergibt 81,2 g (52,3 mmol) 72,3 % der Theorie von der gewünschten Verbindung.

**BEISPIEL 12**

Hydroxymalonsäure-bis[3-(2,3-dihydroxy-N-methyl-propylcarbamoyl)-5-(2,3-dihydroxy-propylcarbamoyl)-2,4,6-triiod-N-methyl-anilid]

a) 5-Methylamino-2,4,6-triiod-isophthalsäure-(2,3-diacetoxypropyl)-monoamidchlorid

122 g (200 mmol) 5-Methylamino-2,4,6-triiod-isophthalsäure-dichlorid werden in 400 ml Dioxan gelöst, 71,5 g (250 mmol) $Na_2CO_3$-Dekahydrat und 18,2 g (200 mmol) 2,3-Dihydroxy-propylamin hinzugefügt

und das Gemisch 3 Stunden bei Raumtemperatur gerührt. Der Niederschlag wird dann abgesaugt, das Filtrat zu einem Schaum eingedampft, dieser in 200 ml Dioxan aufgenommen, 36,75 g (360 mmol) Acetanhydrid und 2,44 g (20 mmol) 4-Dimethylamino-pyridin zugefügt und 3 Stunden bei 80 °C gerührt. Es entsteht eine homogene Lösung. Die Reaktionslösung wird im Vakuum zu einem Schaum eingedampft, dieser in 200 ml Aceton gelöst und an 2 kg Kieselgel 60 (Merck) mit Hexan/Essigester (Essigesteranteil 20->50% linear gesteigert) chromatographiert. Die entsprechenden Fraktionen werden zusammengefaßt und ergeben nach dem Eindampfen 65,12 g (87 mmol) = 43,5% der Theorie der Verbindung als amorphen Feststoff.

b) Benzyloxy-malonsäure-bis[3-chlorcarbonyl-2,4,6-triiod-5-(2,3-diacetoxypropylcarbamoyl)-N-methyl-anilid]

60 g (80 mmol) 5-Methylamino-2,4,6-triiod-isophthalsäure-(2,3-diacetoxypropyl)-monoamid-chlorid werden in 600 ml Toluol suspendiert und die Suspension auf 100 °C erhitzt. Es entsteht eine klare Lösung. Dieser werden 9,88 g (40 mmol) Benzyloxymalonsäuredichlorid (hergestellt analog Hammond et al., Soc. 1957, 1062) zugefügt. Nach wenigen Minuten fällt das Bisanilid als kristalliner Niederschlag aus. Nach 30 Minuten wird die Heizung entfernt, auf Raumtemperatur gekühlt, der Niederschlag abgesaugt und bei 50 °C im Vakuum 24 Stunden getrocknet. Man erhält 48,46 g (29,3 mmol) = 73,2% der Theorie des Bisanilids als Kristallisat. Fp.: > 350 °C

c) Benzyloxy-malonsäure-bis[3-(2,3-diacetoxy-N-methyl-propylcarbamoyl)-5-(2,3-diacetoxy-propylcarbamoyl)-2,4,6-triiod-N-methyl-anilid]

82,76 g (50 mmol) Benzyloxy-malonsäure-bis[3-chlorcarbonyl-2,4,6-triiod-5-(2,3-diacetoxypropylcarbamoyl)-N-methyl-anilid] werden in 830 ml Aceton gelöst, 18,6 g (65 mmol) $Na_2CO_3$ x 10 $H_2O$, 6,3 g (60 mmol) N-Methylamino-propandiol-2,3 hinzugefügt und 2 Stunden am Rückfluß gekocht. Die Umsetzung ist dann vollständig. Es wird auf Raumtemperatur gekühlt, der feste Niederschlag abgesaugt, das Filtrat im Vakuum zum Öl eingeengt, dieses in 300 ml Dioxan gelöst, restliches Wasser durch azeotrope Destillation entfernt, das dabei verbrauchte Dioxan wieder ersetzt, 36,75g (360 mmol) Acetanhydrid und 0.61 g (5 mmol) 4-Dimethylamino-pyridin zugesetzt und 3 Stunden bei 80 °C gerührt. Das Lösungsmittel wird im Vakuum abdestilliert, der ölige Rückstand in Essigester gelöst und an 850 g Kieselgel 60 mit Hexan/Essigester 1:1 chromatographiert. Die entsprechenden Fraktionen werden zusammengefaßt und im Vakuum zu einem festen Schaum eingedampft. Die Ausbeute beträgt 77,1 g (39 mmol) = 78% der Theorie.

d) Hydroxy-malonsäure-bis[3-(2,3-dihydroxy-N-methyl-propylcarbamoyl-5-(2,3-dihydroxy-propylcarbamoyl)-2,4,6-triiod-N-methyl-anilid]

39,5 g (20 mmol) Benzyloxy-malonsäure-bis[3-(2,3-diacetoxy-N-methyl-propylcarbamoyl)-5-(2,3-diacetoxy-propylcarbamoyl)-2,4,6-triiod-N-methyl-anilid] werden in 200 ml absolutem Ethanol gelöst und insgesamt 1,38 g (60 mmol) Natrium in kleinen Portionen zugesetzt. Die Lösung wird bei Raumtemperatur 12 Stunden gerührt. Die Reaktionslösung wird dann im Vakuum auf ungefähr die Hälfte eingeengt, mit 100 ml Wasser versetzt und mit konz. NaOH bei 50 °C bei pH 10-12 verseift. Nach beendeter Verseifung wird mit konz. Salzsäure neutralisiert, im Vakuum zum Öl eingedampft, dieses in 100 ml Wasser gelöst und die Lösung an Ionenaustauschern entsalzt. Das Eluat wird im Vakuum zum Schaum eingedampft und dieser 24 Stunden im Vakuum bei 50 °C getrocknet. Die Ausbeute beträgt 23,25 g (15 mmol) = 75% der Theorie.

**BEISPIEL 13**

Hydroxymalonsäure-bis[3-(2,3-dihydroxy-N-methyl-propylcarbamoyl-5-(2,3-dihydroxy-propylcarbamoyl)-2,4,6-triiod-N-methylanilid]

a) Brommalonsäure-bis[3-chlorcarbonyl-2,4,6-triiod-5-(2,3-diacetoxy-propylcarbamoyl)-N-methyl-anilid]

37,42 g (50 mmol) 5-Methylamino-2,4,6-triiod-isophthalsäure-(2,3-diacetoxypropyl)-monoamid-chlorid werden in 370 ml Toluol suspendiert und die Suspension auf 100 °C erhitzt. Es entsteht eine klare Lösung. Dieser werden 5,5 g (25 mmol) Brommalonsäuredichlorid (Ber. 1908, 4465) zugefügt. Nach wenigen Minuten fällt das Bisanilid als kristalliner Niederschlag aus. Nach 30 Minuten wird die Heizung entfernt, auf Raumtemperatur gekühlt, der Niederschlag abgesaugt und bei 50 °C im Vakuum 24 Stunden getrocknet. Man erhält 31,32 g (19,05 mmol) = 76,2% der Theorie des Bisanilids als Kristallisat. Fp.: > 350 °C

b) Brommalonsäure-bis[3-(2,3-diacetoxy-N-methyl-propylcarbamoyl)-5-(2,3-diacetoxy-propylcarbamoyl)-2,4,6-triiod-N-methyl-anilid]

24,66 g (15 mmol) Brommalonsäure-bis[3-chlorcarbonyl-2,4,6-triiod-5-(2,3-diacetoxy-propylcarbamoyl)-N-methyl-anilid] werden in 250 ml Aceton gelöst, 5,58 g (19,5 mmol) $Na_2CO_3$ x $H_2O$, 1,89 g (18 mmol) N-Methylamino-propandiol-2,3 hinzugefügt und 2 Stunden am Rückfluß gekocht. Es wird auf Raumtemperatur gekühlt, der feste Niederschlag abgesaugt und das Filtrat im Vakuum zum Öl eingeengt. Man löst das Öl in 200 ml Dioxan, entfernt restliches Wasser durch azeotrope Destillation und ersetzt das dabei verbrauchte Dioxan wieder. Man fügt 11,02 g (108 mmol) Acetanhydrid und 0,183 g (1,5 mmol) 4-Dimethylamino-pyridin hinzu und rührt 3 Stunden bei 80 °C. Das Lösungsmittel wird im Vakuum abdestilliert, der ölige Rückstand in Essigester gelöst und an 500 g Kieselgel 60 mit Hexan/Essigester 1:1 chromatographiert. Die entsprechenden Fraktionen werden zusammengefaßt und im Vakuum zu einem festen Schaum eingedampft. Die Ausbeute beträgt 20,94 g (10,74 mmol) = 71,6% der Theorie.

c) Hydroxymalonsäure-bis[3-(2,3-dihydroxy-N-methyl-propylcarbamoyl)-5-(2,3-di-hydroxy-propylcarbamoyl)-2,4,6-triiod-N-methylanilid]

19,5 g (10 mmol) Brommalonsäure-bis[3-(2,3-diacetoxy-N-methyl-propylcarbamoyl)-5-(2,3-diacetoxy-propylcarbamoyl)-2,4,6-triiod-N-methyl-anilid] werden in 50 ml DMF gelöst, 4,91 g (50 mmol) Kaliumacetat hinzugefügt und die Lösung 12 Stunden bei 50 °C gerührt. Die Reaktionslösung wird dann in 500 ml Wasser gefüllt, wobei das intermediäre Peracetat als amorpher Niederschlag ausfällt. Dieser Niederschlag wird abgesaugt, mit Wasser gewaschen, in 100 ml Wasser suspendiert und mit konz. NaOH bei pH 10-12 und 50 °C verseift. Nach beendeter Verseifung wird mit Salzsäure neutralisiert und an Ionenaustauschern entsalzt. Das wäßrige Eluat wird zur Trockne eingedampft. Die Ausbeute beträgt 11,32 g (7,3 mmol) = 73% der Theorie.

## Patentansprüche

## Patentansprüche für folgende Vertragsstaaten : AT, BE, CH, DE, FR, GB, GR, IT, LI, LU, NL, SE

**1.** Dicarbonsäure-bis(3,5-dicarbamoyl-2,4,6-triiodanilide) der allgemeinen Formel I

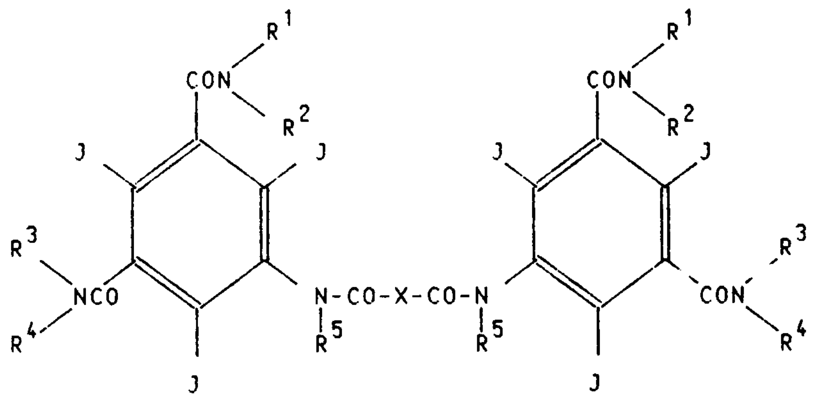

(I)

worin die Amidreste $-CONR^1R^2$ und $-CONR^3R^4$ voneinander verschieden sind und

- $R^1$ ein Wasserstoffatom, einen niederen Alkylrest oder $R^2$
- $R^2$ einen gerad- oder verzweigtkettigen Mono- oder Polyhydroxyalkylrest,
- $R^3$ ein Wasserstoffatom, einen niederen Alkylrest oder $R^4$
- $R^4$ einen gerad- oder verzweigtkettigen Mono- oder Polyhydroxyalkylrest,
- $R^5$ ein Wasserstoffatom, einen niederen Alkylrest oder einen Mono- oder Polyhydroxyalkylrest und
- X ein gerad- oder verzweigtkettiges Alkylen mit 1 bis 6 Kohlenstoffatomen, das gewünschtenfalls durch 1 bis 6 Hydroxy- oder Alkoxygruppen substituiert oder durch ein oder mehrere Sauerstoffatome unterbrochen sein kann,

bedeutet.

**2.** Malonsäure-bis[3-(2,3-dihydroxy-N-methyl-propylcarbamoyl)-5-(2,3-dihydroxy-propylcarbamoyl)-2,4,6-triiod-N-methyl-anilid],
Malonsäure-bis{3-(2,3-dihydroxy-propylcarbamoyl)-5-[bis(2-hydroxyethyl)-carbamoyl])-2,4,6-triiod-N-methyl-anilid},
Malonsäure-bis{3-(2,3-dihydroxy-propylcarbamoyl)-5-[(1RS,2SR)-2,3-dihydroxy-1-hydroxymethylpropyl-carbamoyl]-2,4,6-triiod-N-methyl-anilid},
Malonsäure-bis[3-(2-hydroxy-N-methyl-ethyl-carbamoyl)-5-(2,3-dihydroxy-propylcarbamoyl)-2,4,6-triiod-N-(2-hydroxy-ethyl)-anilid],
Malonsäure-bis[3-(2,3-dihydroxy-N-methyl-propyl-carbamoyl)-5-(2-hydroxyethyl-carbamoyl)-2,4,6-triiod-N-methyl-anilid},
Malonsäure-bis{3-(2,3-dihydroxy-propylcarbamoyl)-5-(2-hydroxyethyl-carbamoyl)-2,4,6-triiod-N-(2,3-dihydroxy-propyl)-anilid],
Hydroxymalonsäure-bis[3-(2,3-dihydroxy-propylcarbamoyl)-5-(2-hydroxy-ethylcarbamoyl)-2,4,6-triiod-N-(2,3-dihydroxy-propyl)-anilid],
Methoxymalonsäure-bis[3-(2-hydroxy-1-hydroxymethylethyl-carbamoyl)-5-(2,3-dihydroxy-propylcarbamoyl)-2,4,6-triiod-N-methyl-anilid],
Hydroxymalonsäure-bis[3-(2-hydroxy-1-hydroxymethylethyl-carbamoyl)-5-(2,3-dihydroxy-propylcarbamoyl)-2,4,6-triiod-N-methyl-anilid],
2,3-Dihydroxy-bernsteinsäue-bis[3-(2,3-dihydroxy-propylcarbamoyl)-5-(2-hydroxy-ethylcarbamoyl)-2,4,6-triiod-N-(2-hydroxyethyl)-anilid],
Hydroxymalonsäure-bis{3-(2,3-dihydroxy-N-methyl-propylcarbamoyl)-5-(2,3-dihydroxy-propylcarbamoyl)-2,4,6-triiod-N-methyl-anilid}.

**3.** Verfahren zur Herstellung der Verbindungen der Formel I, dadurch gekennzeichnet, daß man in an sich bekannter Weise eine Dicarbonsäure-bis[(3-carbamoyl)-(5-chlorcarbonyl)]-2,4,6-triiodanilid der Formel II

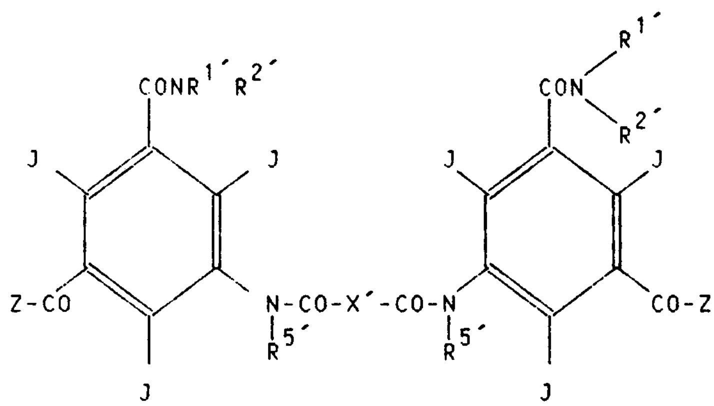

(II)

worin

| | |
|---|---|
| $R^{1'}$ und $R^{2'}$ | die Bedeutung von $R^1$ und $R^2$ besitzen, wobei in $R^1$ und $R^2$ anwesende freie Hydroxylgruppen gegebenenfalls in geschützter Form vorliegen können, |
| X' | die Bedeutung von X oder eines in X umzuwandelnden Substituenten hat |
| $R^{5'}$ | die Bedeutung eines Wasserstoffatoms oder eines niederen Alkylrestes hat und |
| Z | einen reaktiven Säure- oder Esterrest bedeuten, |

mit einer Base der Formel III

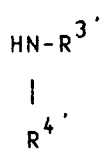

(III)

umsetzt,
worin
$R^{3'}$ und $R^{4'}$ die Bedeutung von $R^3$ und $R^4$ besitzen, wobei in $R^3$ und $R^4$ anwesende freie Hydroxylgruppen gegebenenfalls in geschützter Form vorliegen können und
gegebenenfalls anschließend die aromatischen Acylaminogruppen N-alkyliert und zwar im Falle, daß Verbindungen der allgemeinen Formel I mit $R^5$ gleich niederer Alkylrest gewünscht sind, mit einer Verbindung der allgemeinen Formel IV

$R^5$ -D (IV)

und im Falle, daß Verbindungen der allgemeinen Formel I mit $R^5$ gleich Mono-oder Polyhydroxylrest gewünscht sind, mit einer Verbindung der allgemeinen Formel V

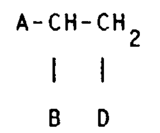

(V)

umsetzt,
worin

- $R^5$ eine niedere Alkylgruppe,
- A ein Wasserstoffatom oder ein Mono- oder Polyhydroxyalkylrest mit 1 bis 4 Kohlenstoffatomen und 1 bis 4 Hydroxylgruppen, darstellen,
- B und D entweder gemeinsam einen Oxidoring bilden oder
- B eine Hydroxygruppe und
- D ein Chlor- oder Bromatom oder eine Sulfat- oder Alkylsulfatgruppe darstellen und im Falle, daß die Gruppe X der Verbindung I eine Methylengruppe bedeutet und alle in der Verbindung I vorhandenen Hydroxylgruppen in geschützter Form vorliegen, gewünschtenfalls mit einem geeigneten hydroxylierenden Reagenz umsetzt, und/oder die Schutzgruppen von geschützten Hydroxygruppen entfernt.

**4.** Röntgenkontrastmittel enthaltend Verbindungen gemäß Anspruch 1 und 2 als schattengebende Substanz.

**Patentansprüche für folgenden Vertragsstaat : ES**

**1.** Verfahren zur Herstellung von Dicarbonsäure-bis(3,5-dicarbamoyl-2,4,6-triiodanilide) der allgemeinen Formel I

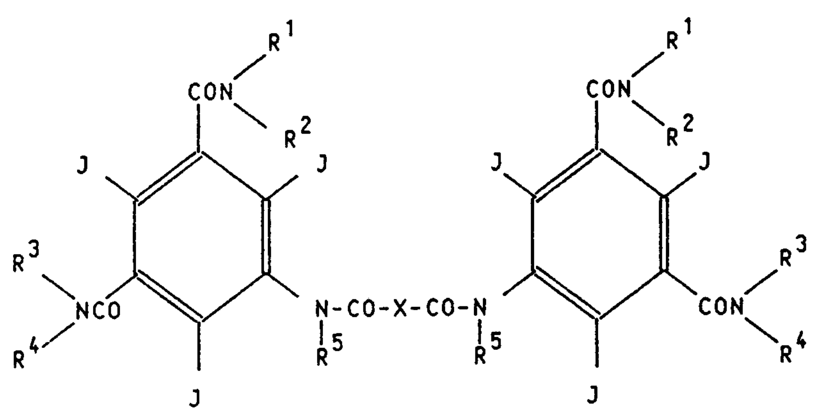

(I)

worin die Amidreste $-CONR^1R^2$ und $-CONR^3R^4$ voneinander verschieden sind und

- $R^1$ ein Wasserstoffatom, einen niederen Alkylrest oder $R^2$
- $R^2$ einen gerad- oder verzweigtkettigen Mono- oder Polyhydroxyalkylrest,
- $R^3$ ein Wasserstoffatom, einen niederen Alkylrest oder $R^4$
- $R^4$ einen gerad- oder verzweigtkettigen Mono- oder Polyhydroxyalkylrest,
- $R^5$ ein Wasserstoffatom, einen niederen Alkylrest oder einen Mono- oder Polyhydroxyalkylrest und
- X ein gerad- oder verzweigtkettiges Alkylen mit 1 bis 6 Kohlenstoffatomen, das gewünschtenfalls durch 1 bis 6 Hydroxy- oder Alkoxygruppen substituiert oder durch ein oder mehrere Sauerstoffatome unterbrochen sein kann,

bedeutet,
dadurch gekennzeichnet, daß man in an sich bekannter Weise eine Dicarbonsäure-bis[(3-carbamoyl)-(5-chlorcarabonyl)]-2,4,6-triiodanilid der Formel II

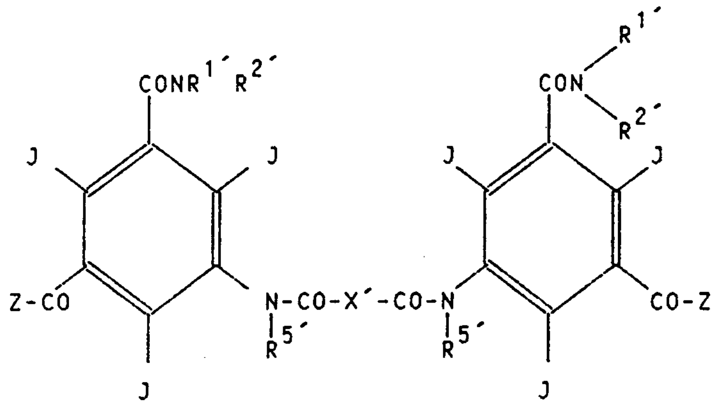

(II)

worin

- $R^{1'}$ und $R^{2'}$ die Bedeutung von $R^1$ und $R^2$ besitzen, wobei in $R^1$ und $R^2$ anwesende freie Hydroxylgruppen gegebenenfalls in geschützter Form vorliegen können,
- X' die Bedeutung von X oder eines in X umzuwandelnden Substituenten hat
- $R^{5'}$ die Bedeutung eines Wasserstoffaatoms oder eines niederen Alkylrestes hat und
- Z einen reaktiven Säure- oder Esterrest bedeuten,

mit einer Base der Formel III

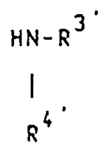

(III)

umsetzt.
worin
$R^{3'}$ und $R^{4'}$ die Bedeutung von $R^3$ und $R^4$ besitzen, wobei in $R^3$ und $R^4$ anwesende freie Hydroxylgruppen gegebenenfalls in geschützter Form vorliegen können und
gegebenenfalls anschließend die aromatischen Acylaminogruppen N-alkyliert und zwar im Falle, daß Verbindungen der allgemeinen Formel I mit $R^5$ gleich niederer Alkylrest gewünscht sind, mit einer Verbindung der allgemeinen Formel IV

$R^5$ -D (IV)

und im Falle, daß Verbindungen der allgemeinen Formel I mit $R^5$ gleich Mono-oder Polyhydroxylrest gewünscht sind, mit einer Verbindung der allgemeinen Formel V

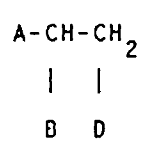

(V)

umsetzt,
worin

- $R^5$ eine niedere Alkylgruppe,
- A ein Wasserstoffatom oder ein Mono- oder Polyhydroxyalkylrest mit 1 bis 4 Kohlenstoffatomen und 1 bis 4 Hydroxylgruppen darstellen,
- B und D entweder gemeinsam einen Oxidoring bilden oder
- B eine Hydroxygruppe und
- D ein Chlor- oder Bromatom oder eine Sulfat- oder Alkylsulfatgruppe darstellen und im Falle, daß die Gruppe X der Verbindung I eine Methylengruppe bedeutet und alle in der Verbindung

I vorhandenenen Hydroxylgruppen in geschützter Form vorliegen, gewünschtenfalls mit einem geeigneten hvdroxylierenden Reagenz umsetzt,
und/oder die Schutzgruppen von geschützten Hydroxygruppen entfernt.

**2.** Verfahren nach Anspruch 1, dadurch gekennzeichnet, daß als Dicarbonsäure-bis(3,5-dicarbamoyl-2,4,6-triiodanilid)
Malonsäure-bis[3-(2,3-dihydroxy-N-methyl-propylcarbamoyl)-5-(2,3-dihydroxypropylcarbamoyl)-2,4,6-triiod-N-methyl-anilid],
Malonsäure-bis{3-(2,3-dihydroxy-propylcarbamoyl)-5-[bis(2-hydroxyethyl)-carbamoyl]-2,4,6-triiod-N-methyl-anilid},
Malonsäure-bis{3-(2,3-dihydroxy-propylcarbamoyl)-5[(1RS,2SR)-2,3-dihydroxy-1-hydroxymethylpropyl-carbamoyl]-2,4,6-triiod-N-methyl-anilid},
Malonsäure-bis[3-(2-hydroxy-N-methyl-ethyl-carbamoyl)-5-(2,3-dihydroxypropylcarbamoyl)-2,4,6-triiod-N-(2-hydroxy-ethyl)-anilid],
Malonsäure-bis[3-(2,3-dihydroxy-N-methyl-propyl-carbamoyl)-5-(2-hydroxyethyl-carbamoyl)-2,4,6-triiod-N-methyl-anilid],
Malonsäure-bis{3-(2,3-dihydroxy-propylcarbamoyl)-5-(2-hydroxyethyl-carbamoyl)-2,4,6-triiod-N-(2,3-dihydroxy-propyl)-anilid},
Hydroxymalonsäure-bis[3-(2,3-dihydroxy-propylcarbamoyl)-5-(2-hydroxy-ethylcarbamoyl)-2,4,6-triiod-N-(2,3-dihydroxy-propyl)-anilid],
Hydroxymalonsäure-bis[3-(2-hydroxy-1-hydroxymethylethyl-carbamoyl)-5-(2,3-dihydroxy-propyl-carbamoyl)-2,4,6-triiod-N-methyl-anilid],
Methoxymalonsäure-bis[3-(2-hydroxy-1-hydroxymethylethyl-carbamoyl)-5-(2,3-dihydroxy-propyl-carbamoyl)-2,4,6-triiod-N-methyl-anilid],
2,3-Dihydroxy-bernsteinsäue-bis[3-(2,3-dihydroxy-propylcarbamoyl)-5-(2-hydroxy-ethylcarbamoyl)-2,4,6-triiod-N-(2-hydroxyethyl)-anilid],
Hydroxymalonsäure-bis{3-(2,3-dihydroxy-N-methyl-propylcarbamoyl)-5-(2,3-dihydroxy-propylcarbamoyl)-2,4,6-triiod-N-methyl-anilid}
hergestellt wird.

## Claims
**Claims for the following Contracting States : AT, BE, CH, DE, FR, GB, GR, IT, LI, LU, NL, SE**

**1.** Dicarboxylic acid bis(3,5-dicarbamoyl-2,4,6-triiodoanilides) of the general formula I

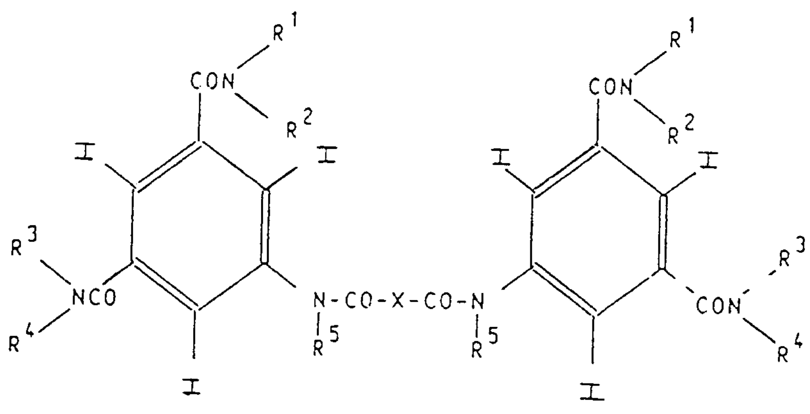

(I)

in which the amide radicals -CONR[1]R[2] and -CONR[3]R[4] are different from one another and

- $R^1$ represents a hydrogen atom, a lower alkyl radical or $R^2$,
- $R^2$ represents a straight-chain or branched-chain mono- or poly-hydroxyalkyl radical,
- $R^3$ represents a hydrogen atom, a lower alkyl radical or $R^4$,
- $R^4$ represents a straight-chain or branched-chain mono- or poly-hydroxyalkyl radical,
- $R^5$ represents a hydrogen atom, a lower alkyl radical or a mono- or poly-hydroxyalkyl radical, and
- X represents a straight-chain or branched-chain alkylene radical having from 1 to 6 carbon

atoms which, if desired, may be substituted by from 1 to 6 hydroxy or alkoxy groups or interrupted by one or more oxygen atoms.

**2.** Malonic acid bis[3-(2,3-dihydroxy-N-methylpropylcarbamoyl)-5-(2,3-dihydroxypropylcarbamoyl)-2,4,6-triiodo-N-methylanilide],
malonic acid bis{3-(2,3-dihydroxypropylcarbamoyl)-5-[bis(2-hydroxyethyl)carbamoyl]-2,4,6-triiodo-N-methylanilide},
malonic acid bis{3-(2,3-dihydroxypropylcarbamoyl)-5-[(1RS,2SR)-2,3-dihydroxy-1-hydroxymethylpropylcarbamoyl]-2,4,6-triiodo-N-methylanilide},
malonic acid bis[3-(2-hydroxy-N-methylethylcarbamoyl)-5-(2,3-dihydroxypropylcarbamoyl)-2,4,6-triiodo-N-(2-hydroxyethyl)anilide],
malonic acid bis[3-(2,3-dihydroxy-N-methylpropylcarbamoyl)-5-(2-hydroxyethylcarbamoyl)-2,4,6-triiodo-N-methylanilide],
malonic acid bis{3-(2,3-dihydroxypropylcarbamoyl)-5-(2-hydroxyethylcarbamoyl)-2,4,6-triiodo-N-(2,3-dihydroxypropyl)anilide},
hydroxymalonic acid bis[3-(2,3-dihydroxypropylcarbamoyl)-5-(2-hydroxyethylcarbamoyl)-2,4,6-triiodo-N-(2,3-dihydroxypropyl)anilide],
hydroxymalonic acid bis[3-(2-hydroxy-1-hydroxymethylethylcarbamoyl)-5-(2,3-dihydroxypropylcarbamoyl)-2,4,6-triiodo-N-methylanilide],
methoxymalonic acid bis[3-(2-hydroxy-1-hydroxymethylethylcarbamoyl)-5-(2,3-dihydroxypropylcarbamoyl)-2,4,6-triiodo-N-methylanilide],
2,3-dihydroxysuccinic acid bis[3-(2,3-dihydroxypropylcarbamoyl)-5-(2-hydroxyethylcarbamoyl)-2,4,6-triiodo-N-(2-hydroxyethyl)anilide],
hydroxymalonic acid bis(3-(2,3-dihydroxy-N-methylpropylcarbamoyl)-5-(2,3-dihydroxypropylcarbamoyl)-2,4,6-triiodo-N-methylanilide).

**3.** Process for the preparation of the compounds of formula I, characterised in that, in a manner known per se, a dicarboxylic acid bis[(3-carbamoyl)-(5-chlorocarbonyl)]-2,4,6-triiodoanilide of formula II

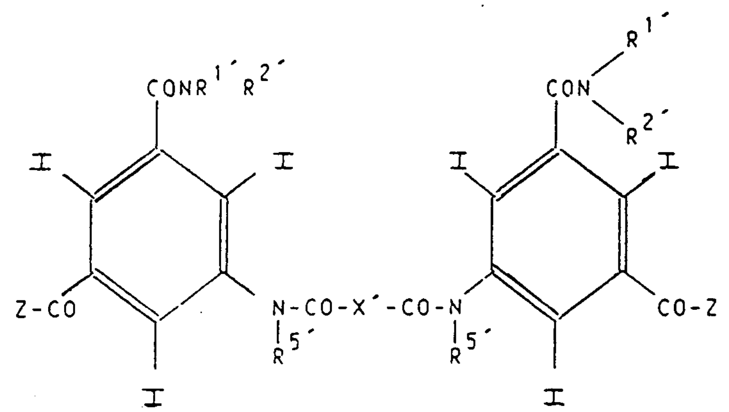

(II)

in which
$R^{1'}$ and $R^{2'}$ have the meaning of $R^1$ and $R^2$, and free hydroxy groups present in $R^1$ and $R^2$ may optionally be in protected form,
X' has the meaning of X or of a substituent to be converted into X,
$R^{5'}$ represents a hydrogen atom or a lower alkyl radical and
Z represents a reactive acid radical or ester radical,
is reacted with a base of formula III

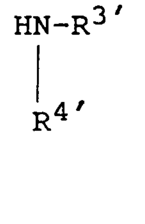

(III)

in which
$R^{3'}$ and $R^{4'}$ have the meaning of $R^3$ and $R^4$, and free hydroxy groups present in $R^3$ and $R^4$ may optionally be in protected form and.
then optionally the aromatic acylamino groups are N-alkylated, namely as follows: in the case where compounds of the general formula I in which $R^5$ represents a lower alkyl radical are desired, by reaction with a compound of the general formula IV

$R^5$-D (IV)

and in the case where compounds of the general formula I in which $R^5$ represents a mono- or poly-hydroxy radical are desired, by reaction with a compound of the general formula V

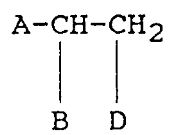

(V)

in which
$R^5$ represents a lower alkyl group,
A represents a hydrogen atom or a mono- or poly-hydroxyalkyl radical having from 1 to 4 carbon atoms and from 1 to 4 hydroxy groups,
B and D either together form an oxido ring or
B represents a hydroxy group and
D represents a chlorine or bromine atom or a sulphate group or an alkyl sulphate group;
and in the case where the group X of compound I represents a methylene group and all hydroxy groups present in compound I are in protected form, reaction is, if desired, carried out with a suitable hydroxylating reagent,
and/or the protecting groups of protected hydroxy groups are removed.

**4.** X-ray contrast media containing compounds according to claims 1 and 2 as radio-opaque substance.

**Claims for the following Contracting State : ES**

**1.** Process for the preparation of dicarboxylic acid bis(3,5-dicarbamnoyl-2,4,6-triiodo-anilides) of the general formula I

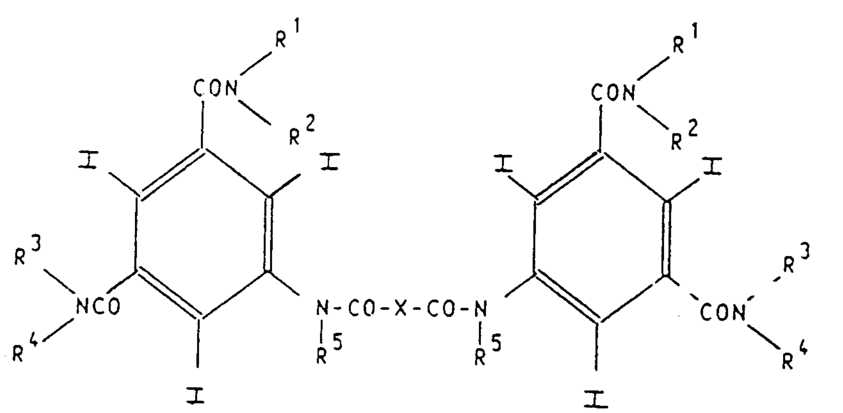

(I)

in which the amide radicals $-CONR^1R^2$ and $-CONR^3R^4$ are different from one another and
$R^1$ represents a hydrogen atom, a lower alkyl radical or $R^2$,
$R^2$ represents a straight-chain or branched-chain mono- or poly-hydroxyalkyl radical,
$R^3$ represents a hydrogen atom, a lower alkyl radical or $R^4$,
$R^4$ represents a straight-chain or branched-chain mono- or poly-hydroxyalkyl radical,
$R^5$ represents a hydrogen atom, a lower alkyl radical or a mono- or poly-hydroxyalkyl radical,

and

X represents a straight-chain or branched-chain alkylene radical having from 1 to 6 carbon atoms which, if desired, may be substituted by from 1 to 6 hydroxy or alkoxy groups or interrupted by one or more oxygen atoms,

characterised in that, in a manner known per se, a dicarboxylic acid bis[(3-carbamoyl)-(5-chlorocarbonyl)]-2,4,6-triiodoanilide of formula II

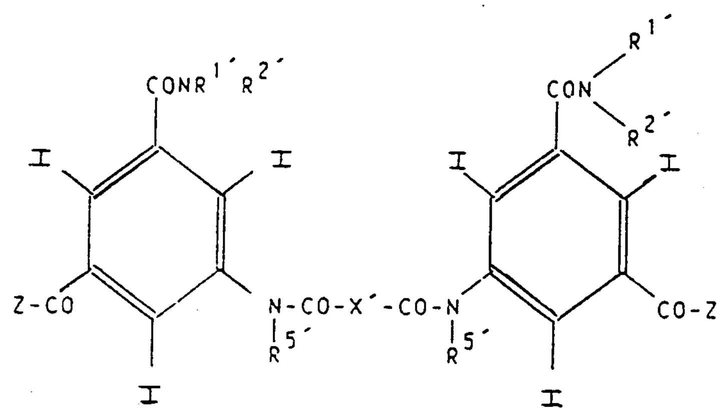

(II)

in which
$R^{1'}$ and $R^{2'}$ have the meaning of $R^1$ and $R^2$, and free hydroxy groups present in $R^1$ and $R^2$ may optionally be in protected form,
X' has the meaning of X or of a substituent to be converted into X,
$R^{5'}$ represents a hydrogen atom or a lower alkyl radical and
Z represents a reactive acid radical or ester radical,
is reacted with a base of formula III

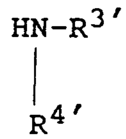

(III)

in which
$R^{3'}$ and $R^{4'}$ have the meaning of $R^3$ and $R^4$, and free hydroxy groups present in $R^3$ and $R^4$ may optionally be in protected form and
then optionally the aromatic acylamino groups are N-alkylated, namely as follows: in the case where compounds of the general formula I in which $R^5$ represents a lower alkyl radical are desired, by reaction with a compound of the general formula IV

$R^5$-D (IV)

and in the case where compounds of the general formula I in which $R^5$ represents a mono- or polyhydroxy radical are desired, by reaction with a compound of the general formula V

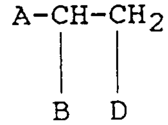

(V)

in which
$R^5$ represents a lower alkyl group,
A represents a hydrogen atom or a mono- or poly-hydroxyalkyl radical having from 1 to 4 carbon atoms and from 1 to 4 hydroxy groups,

B and D either together form an oxido ring or
B represents a hydroxy group and
D represents a chlorine or bromine atom or a sulphate group or an alkyl sulphate group;
and in the case where the group X of compound I represents a methylene group and all hydroxy groups present in compound I are in protected form, reaction is, if desired, carried out with a suitable hydroxylating reagent,
and/or the protecting groups of protected hydroxy groups are removed.

**2.** Process according to claim 1, characterized in that, as dicarboxylic acid bis(3,5-dicarbamoyl-2,4,6-triiodoanilides)
malonic acid bis[3-(2,3-dihydroxy-N-methylpropylcarbamoyl)-5-(2,3-dihydroxypropylcarbamoyl)-2,4,6-triiodo-N-methylanilide],
malonic acid bis{3-(2,3-dihydroxypropylcarbamoyl)-5-[bis(2-hydroxyethyl)carbamoyl]-2,4,6-triiodo-N-methylanilide},
malonic acid bis{3-(2,3-dihydroxypropylcarbamoyl)-5-[(1RS,2SR)-2,3-dihydroxy-1-hydroxymethylpropylcarbamoyl]-2,4,6-triiodo-N-methylanilide},
malonic acid bis[3-(2-hydroxy-N-methylethylcarbamoyl)-5-(2,3-dihydroxypropylcarbamoyl)-2,4,6-triiodo-N-(2-hydroxyethyl)anilide],
malonic acid bis[3-(2,3-dihydroxy-N-methylpropylcarbamoyl)-5-(2-hydroxyethylcarbamoyl)-2,4,6-triiodo-N-methylanilide],
malonic acid bis{3-(2,3-dihydroxypropylcarbamoyl)-5-(2-hydroxyethylcarbamoyl)-2,4,6-triiodo-N-(2,3-dihydroxypropyl)anilide},
hydroxymalonic acid bis[3-(2,3-dihydroxypropylcarbamoyl)-5-(2-hydroxyethylcarbamoyl)-2,4,6-triiodo-N-(2,3-dihydroxypropyl)anilide],
hydroxymalonic acid bis[3-(2-hydroxy-1-hydroxymethylethylcarbamoyl)-5-(2,3-dihydroxypropylcarbamoyl)-2,4,6-triiodo-N-methylanilide],
methoxymalonic acid bis[3-(2-hydroxy-1-hydroxymethylethylcarbamoyl)-5-(2,3-dihydroxypropylcarbamoyl)-2,4,6-triiodo-N-methylanilide],
2,3-dihydroxysuccinic acid bis[3-(2,3-dihydroxypropylcarbamoyl)-5-(2-hydroxyethylcarbamoyl)-2,4,6-triiodo-N-(2-hydroxyethyl)anilide],
hydroxymalonic acid bis{3-(2,3-dihydroxy-N-methylpropylcarbamoyl)-5-(2,3-dihydroxypropylcarbamoyl)-2,4,6-triiodo-N-methylanilide}
are prepared.

## Revendications

**Revendications pour les Etats contractants suivants : AT, BE, CH, DE, FR, GB, GR, IT, LI, LU, NL, SE**

**1.** Bis(3,5-dicarbamoyl-2,4,6-triiodoanilides) d'acides dicarboxyliques de formule générale I ci-dessous

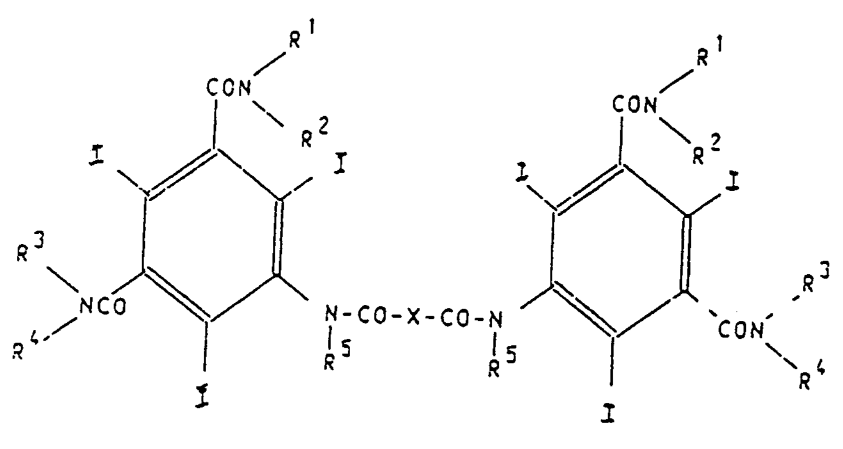

(I)

dans laquelle les radicaux amides $-CONR^1R^2$ et $-CONR^3R^4$ sont différents l'un de l'autre et

- $R^1$ désigne un atome d'hydrogène, un alkyle inférieur ou un radical $R^2$ tel que ci-dessous défini,
- $R^2$ un radical mono- ou poly-hydroxyalkyle à chaîne linéaire ou ramifiée,
- $R^3$ un atome d'hydrogène, un alkyle inférieur ou un radical $R^4$ tel que ci-dessous défini,

R4 un radical mono- ou poly-hydroxyalkyle à chaîne linéaire ou ramifiée,
R5 un atome d'hydrogène, un alkyle inférieur ou un radical mono- ou poly-hydroxyalkyle et
X un alkylène linéaire ou ramifié ayant de 1 à 6 atomes de carbone, éventuellement pouvant avoir comme substituants de 1 à 6 groupes hydroxy ou alcoxy ou pouvant être interrompu par un ou plusieurs atomes d'oxygène.

**2.** Malon-bis[3-(2,3-dihydroxy-N-méthyl-propylcarbamoyl)-5-(2,3-dihydroxy-propylcarbamoyl)-2,4,6-triiodo-N-méthylanilide],

Malon-bis{3-(2,3-dihydroxy-propylcarbamoyl)-5-[bis(2-hydroxyéthyl)-carbamoyl]-2,4,6-triiodo-N-méthyl-anilide},

Malon-bis{3-(2,3-dihydroxy-propylcarbamoyl)-5-[(1RS,2RS)-2,3-dihydroxy-1-hydroxyméthylpropyl-carbamoyl]-2,4,6-triiodo-N-méthyl-anilide},

Malon-bis[3-(2-hydroxy-N-méthyl-éthyl-carbamoyl)-5-(2,3-dihydroxy-propylcarbamoyl)-2,4,6-triiodo-N-(2-hydroxy-éthyl)-anilide],

Malon-bis[3-(2,3-dihydroxy-N-méthyl-propylcarbamoyl)-5-(2-hydroxy-éthyl-carbamoyl)-2,4,6-triiodo-N-méthyl-anilide],

Malon-bis{3-(2,3-dihydroxy-propylcarbamoyl)-5-(2-hydroxyéthyl-carbamoyl)-2,4,6-triiodo-N-(2,3-dihydroxy-propyl)-anilide},

Hydroxymalon-bis[3-(2,3-dihydroxy-propylcarbamoyl)-5-(2-hydroxy-éthyl-carbamoyl)-2,4,6-triiodo-N-(2,3-dihydroxypropyl)-anilide],

Hydroxymalon-bis[3-(2-hydroxy-1-hydroxyméthyléthylcarbamoyl)-5-(2,3-dihydroxy-propyl-carbamoyl)-2,4,6-triiodo-N-méthyl-anilide],

Méthoxymalon-bis[3-(2-hydroxy-1-hydroxyméthyléthylcarbamoyl)-5-(2,3-dihydroxy-propyl-carbamoyl)-2,4,6-triiodo-N-méthyl-anilide],

2,3-Dihydroxy-succin-bis[3-(2,3-dihydroxy-propylcarbamoyl)-5-(2-hydroxy-éthylcarbamoyl)-2,4,6-triiodo-N-(2-hydroxyéthyl)-anilide],

Hydroxymalon-bis{3-(2,3-dihydroxy-N-méthyl-propylcarbamoyl)-5-(2,3-dihydroxy-propylcarbamoyl)-2,4,6-triiodo-N-méthyl-anilide}.

**3.** Procédé de préparation des composés de formule I selon la revendication 1, procédé caractérisé en ce que l'on fait réagir d'une manière en elle-même connue un bis[(3-carbamoyl)-(5-chlorocarbonyl)]-2,4,6-triiodoanilide d'acide dicarboxylique de formule II

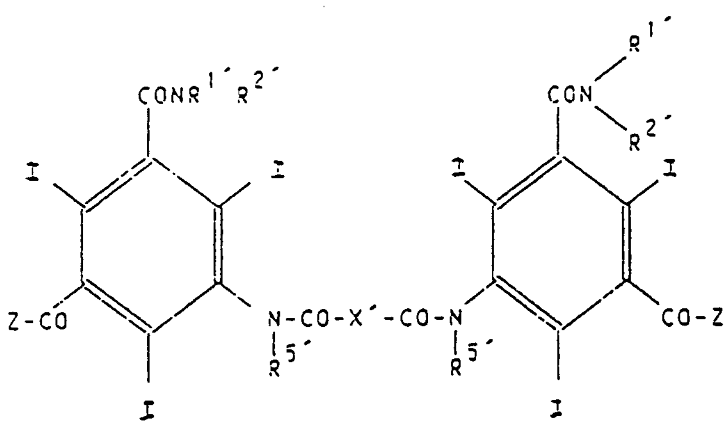

(II)

(dans laquelle

$R^{1'}$ et $R^{2'}$ ont les significations de $R^1$ et $R^2$, des groupes hydroxyliques libres de $R^1$ et $R^2$ pouvant être éventuellement protégés,
X' a la signification de X ou est un substituant à transformer en radical X,
$R^{5'}$ désigne un atome d'hydrogéne ou un alkyle inférieur et
Z un radical d'acide ou d'ester réactif)

avec une base de formule III

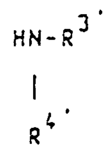

(III)

(dans laquelle
$R^{3'}$ et $R^{4'}$ ont les significations de $R^3$ et $R^4$, des groupes hydroxyliques libres de $R^3$ et $R^4$ pouvant être éventuellement protégés), et
le cas échéant on alkyle ensuite sur l'azote les groupes acylamino aromatiques, à savoir, si l'on veut obtenir des composés de formule I dont $R^5$ est un alkyle inférieur, on fait réagir avec un composé de formule IV

$R^5$-D (IV)

et si l'on veut obtenir des composés dont $R^5$ est un radical mono- ou poly-hydroxyalkyle, on fait réagir avec un composé de formule V

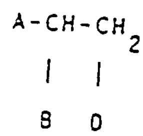

(V)

(formules dans lesquelles
$R^5$ désigne un alkyle inférieur,
A un atome d'hydrogène ou un radical mono- ou poly-hydroxyalkyle ayant de 1 à 4 atomes de carbone et de 1 à 4 hydroxyles,
B et D ou bien forment ensemble un cycle oxido ou bien
B est un hydroxyle et
D un atome de chlore ou de brome ou un groupe sulfate ou alkylsulfate),
et si le groupe X du composé I est un groupe méthylène et que tous les groupes hydroxyliques du composé I sont à l'état protégé, si l'on veut on fait réagir avec un réactif hydroxylant approprié et/ou on élimine les groupes protecteurs des groupes hydroxyliques protégés.

**4.** Produits de contraste pour rayons X contenant comme agents de contraste des composés des revendications 1 et 2.

**Revendications pour l'Etat contractant suivant : ES**

**1.** Procéde de préparation des bis(3,5-dicarbamoyl-2,4,6-triiodoanilides) d'acides dicarboxyliques de formule générale I ci-dessous

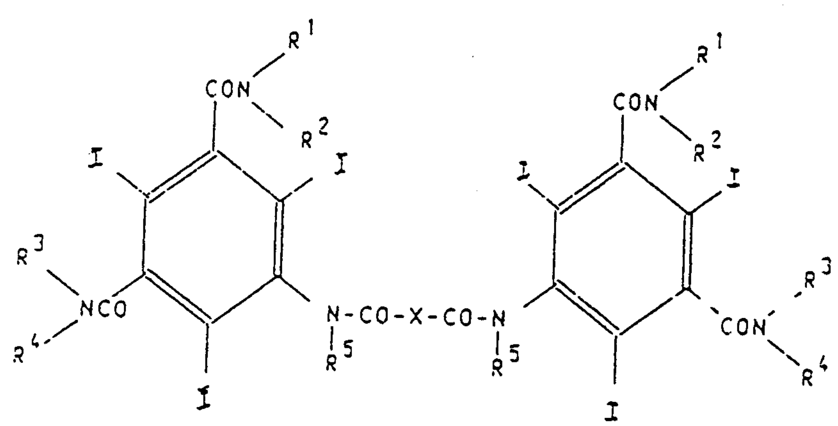

(I)

dans laquelle les radicaux amides $-CONR^1R^2$ et $-CONR^3R^4$ sont différents l'un de l'autre et

- $R^1$ désigne un atome d'hydrogène, un alkyle inférieur ou un radical $R^2$ tel que ci-dessous défini,
- $R^2$ un radical mono- ou poly-hydroxyalkyle à chaîne linéaire ou ramifiée,
- $R^3$ un atome d'hydrogène, un alkyle inférieur ou un radical $R^4$ tel que ci-dessous défini,
- R4 un radical mono- ou poly-hydroxyalkyle à chaîne linéaire ou ramifiée,
- R5 un atome d'hydrogène, un alkyle inférieur ou un radical mono- ou poly-hydroxyalkyle et
- X un alkylène linéaire ou ramifié ayant de 1 à 6 atomes de carbone, éventuellement pouvant avoir comme substituants de 1 à 6 groupes hydroxy ou alcoxy ou pouvant être interrompu par un ou plusieurs atomes d'oxygène,

procédé caractérisé en ce que l'on fait réagir d'une manière en elle-même connue un bis[(3-carbamoyl)- (5-chlorocarbonyl)]-2,4,6-triiodoanilide d'acide dicarboxylique de formule II

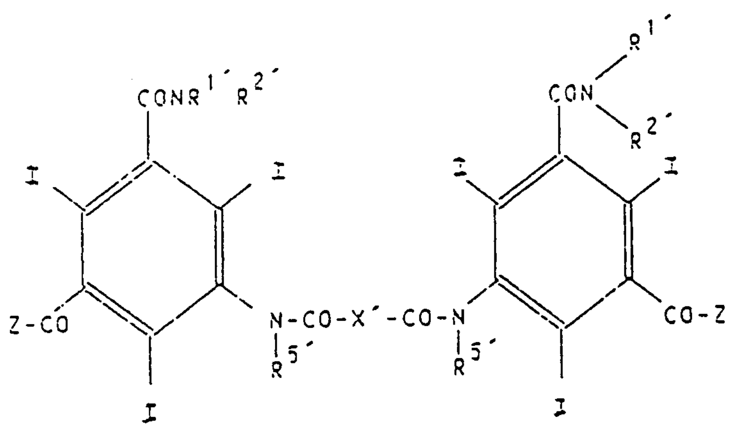

(II)

(dans laquelle

- $R^{1'}$ et $R^{2'}$ ont les significations de $R^1$ et $R^2$, des groupes hydroxyliques libres de $R^1$ et $R^2$ pouvant être éventuellement protégés,
- X' a la signification de X ou est un substituant à transformer en radical X,
- $R^{5'}$ désigne un atome d'hydrogène ou un alkyle inférieur et
- Z un radical d'acide ou d'ester réactif)

avec une base de formule III

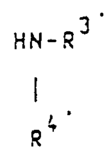

(III)

(dans laquelle
$R^{3'}$ et $R^{4'}$ ont les significations de $R^3$ et $R^4$, des groupes hydroxyliques libres de $R^3$ et $R^4$ pouvant être éventuellement protégés), et
le cas échéant on alkyle ensuite sur l'azote les groupes acylamino aromatiques, à savoir, si l'on veut obtenir des composés de formule I dont $R^5$ est un alkyle inférieur, on fait réagir avec un composé de formule IV

$R^5$-D (IV)

et si l'on veut obtenir des composés dont $R^5$ est un radical mono- ou poly-hydroxyalkyle, on fait réagir avec un composé de formule V

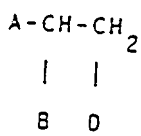

(V)

(formules dans lesquelles
$R^5$ désigne un alkyle inférieur,
A un atome d'hydrogène ou un radical mono- ou poly-hydroxyalkyle ayant de 1 à 4 atomes de carbone et de 1 à 4 hydroxyles,
B et D ou bien forment ensemble un cycle oxido ou bien
B est un hydroxyle et
D un atome de chlore ou de brome ou un groupe sulfate ou alkylsulfate),
et si le groupe X du composé I est un groupe méthylène et que tous les groupes hydroxyliques du composé I sont à l'état protégé, si l'on veut on fait réagir avec un réactif hydroxylant approprié et/ou on élimine les groupes protecteurs des groupes hydroxyliques protégés.

**2.** Procédé de préparation selon la revendication 1, procédé caractérisé en ce que comme des bis(3,5-dicarbamoyl-2,4,6-triiodoanilides) d'acides dicarboxyliques
Malon-bis[3-(2,3-dihydroxy-N-méthyl-propylcarbamoyl)-5-(2,3-dihydroxy-propylcarbamoyl)-2,4,6-triiodo-N-méthylanilide],
Malon-bis{3-(2,3-dihydroxy-propylcarbamoyl)-5-[bis(2-hydroxyéthyl)-carbamoyl]-2,4,6-triiodo-N-méthyl-anilide},
Malon-bis{3-(2,3-dihydroxypropylcarbamoyl)-5-[(1RS,2RS)-2,3-dihydroxy-1-hydroxyméthylpropyl-carbamoyl]-2,4,6-triiodo-N-méthyl-anilide},
Malon-bis[3-(2-hydroxy-N-méthyl-éthyl-carbamoyl)-5-(2,3-dihydroxy-propylcarbamoyl)-2,4,6-triiodo-N-(2-hydroxy-éthyl)-anilide],
Malon-bis[3-(2,3-dihydroxy-N-méthyl-propyl-carbamoyl)-5-(2-hydroxy-éthyl-carbamoyl)-2,4,6-triiodo-N-méthyl-anilide],
Malon-bis{3-(2,3-dihydroxy-propylcarbamoyl)-5-(2-hydroxyéthyl-carbamoyl)-2,4,6-triiodo-N-(2,3-dihydroxy-propyl)-anilide},
Hydroxymalon-bis[3-(2,3-dihydroxy-propylcarbamoyl)-5-(2-hydroxy-éthyl-carbamoyl)-2,4,6-triiodo-N-(2,3-dihydroxypropyl)-anilide],
Hydroxymalon-bis[3-(2-hydroxy-1-hydroxyméthyléthylcarbamoyl)-5-(2,3-dihydroxy-propyl-carbamoyl)-2,4,6-triiodo-N-méthyl-anilide],
Méthoxymalon-bis[3-(2-hydroxy-1-hydroxyméthyléthylcarbamoyl)-5-(2,3-dihydroxy-propyl-carbamoyl)-2,4,6-triiodo-N-méthyl-anilide],
2,3-Dihydroxy-succin-bis[3-(2,3-dihydroxy-propylcarbamoyl)-5-(2-hydroxy-éthylcarbamoyl)-2,4,6-triiodo-N-(2-hydroxyéthyl)-anilide],
Hydroxymalon-bis{3-(2,3-dihydroxy-N-méthyl-propylcarbamoyl)-5-(2,3-dihydroxy-propylcarbamoyl)-2,4,6-triiodo-N-méthyl-anilide}
sont préparés.